(19) Europäisches Patentamt
European Patent Office
Office européen des brevets

(11) Veröffentlichungsnummer : **0 217 092 B1**

(12) # EUROPÄISCHE PATENTSCHRIFT

(45) Veröffentlichungstag der Patentschrift : **28.03.90**

(51) Int. Cl.[5] : **C 07 D309/30**, C 07 D309/10, C 07 F 7/18, C 07 C 69/732, A 61 K 31/365, C 07 D409/10

(21) Anmeldenummer : **86111384.3**

(22) Anmeldetag : **18.08.86**

(54) **3-Desmethyl-mevalonsäurederivate, Verfahren zu ihrer Herstellung, pharmazeutische Präparate auf Basis dieser Verbindungen, ihre Verwendung sowie Zwischenprodukte.**

(30) Priorität : **29.08.85 DE 3530797**
**07.05.86 DE 3615446**

(43) Veröffentlichungstag der Anmeldung : **08.04.87 Patentblatt 87/15**

(45) Bekanntmachung des Hinweises auf die Patenterteilung : **28.03.90 Patentblatt 90/13**

(84) Benannte Vertragsstaaten :
**AT BE CH DE FR GB IT LI LU NL SE**

(56) Entgegenhaltungen :
**EP-A- 0 164 049**
**US-A- 4 440 927**
**CHEMICAL ABSTRACTS, Band 99, 1983, Seite 515, Zusammenfassung Nr. 38364d, Columbus, Ohio, US; & JP-A-58 08076 (MERCK AND CO., INC.) 18-01-1983**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Beck, Gerhard, Dr.**
**Gustav-Freytag-Strasse 24**
**D-6000 Frankfurt am Main (DE)**
Erfinder : **Kerekjarto, Bela, Dr.**
**Weilbächer Wälder FA 8**
**D-6238 Hofheim am Taunus 6 (DE)**
Erfinder : **Lau, Hans-Hermann, Dr.**
**Kastanienhain 29**
**D-6232 Bad Soden am Taunus (DE)**
Erfinder : **Wess, Günther, Dr.**
**Hainstrasse 35**
**D-6455 Erlensee (DE)**

**EP 0 217 092 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Derivate der 3-Hydroxy-3-methyl-glutarsäure (HMG) und der Mevalonsäure sind als Hemmer der Cholesterinbiosynthese beschrieben worden (M. T. Boots et al., J. Pharm. Sci. 69, 306 (1980), F. M. Singer et al., Proc. Soc. Exper. Biol. Med. 102, 270 (1959), H. Feres, Tetrahedron Lett. 24, 3769 (1983)). 3-Hydroxy-3-methyl-glutarsäure selbst zeigt an der Ratte und im Humanversuch signifikante cholesterinsenkende Wirkung (Z. Beg, Experimentia 23, 380 (1967), ibid 24, 15 (1968), P. J. Lupien et al., Lancet 1978, 1, 283)).

Endo et al. (Febs. Letter 72, 323 (1976), J. Biol. Chem. 253, 1121 (1978)) berichteten über die Hemmung der 3-Hydroxy-3-methyl-glutaryl-coenzym-A-Reduktase (HMG-CoA-Reduktase), des geschwindigkeitsbestimmenden Enzyms der Cholesterinbiosynthese, durch das Fermentationsprodukt « Compactin ». Brown et al. (J. Chem. Soc. 1165 (1976)) bestimmten die chemische Struktur und die absolute Konfiguration des « Compactins » durch eine Kombination chemischer, spektroskopischer und radiokristallographischer Methoden und konnten zeigen, daß « Compactin » ein Derivat des 3-Desmethylmevalonsäurelactons ist.

Compactin-Derivate, die die Aktivität der HMG-CoA-Reduktase hemmen, wurden bereits beschrieben (G. E. Stokker et al., J. Med. Chem. 28, 347-358 (1985)).

In der US-Patentschrift 4,440,927 sowie in der europäischen Patentanmeldung A-0 164 049 werden Compactin-Analoga beschrieben, die die in der nachstehend angegebenen Formel I angegebene Grundstruktur aufweisen. Sie tragen in 2-Stellung am Aromaten jedoch keinen über eine $CH_2$— oder $CH_2$—$CH_2$-Brücke gebundenen Cycloalkylrest. Über ihre Hemmwirkung gegeüber der HMG-CoA-Reduktase fehlen exakte Angaben.

Die vorliegende Erfindung betrifft neue synthetische Analoga des « Compactins » der allgemeinen Formel I,

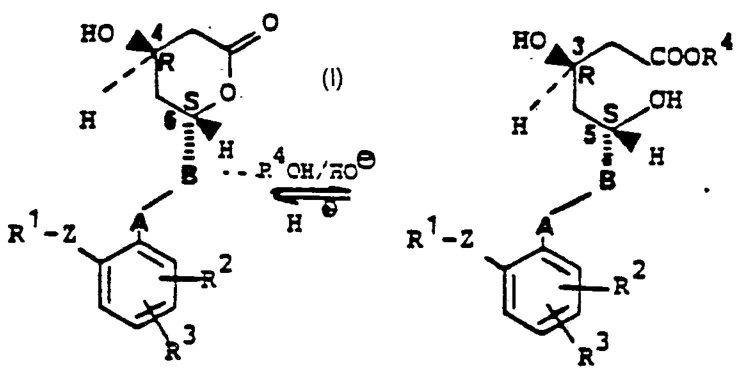


(I)

(Ia)

in Form des dargestellten δ-Lacton der Formel I oder in Form des Dihydroxysäure-Derivates Ia. In den Formeln bedeuten :

A-B einen Rest der Formel —CH=CH— oder —$CH_2$—$CH_2$—,

Z eine —$CH_2$— oder —$CH_2$—$CH_2$— Gruppe,

$R^1$ einen cycloaliphatischen Kohlenwasserstoffrest mit 3 bis 7 Kohlenstoffatomen, der gegebenenfalls mit 1 oder 2 Methylgruppen substituiert ist,

$R^2$, $R^3$ Wasserstoff, Halogen, Trifluoromethyl, Alkyl oder Alkoxy mit je 1-6 C-Atomen,

$R^4$ Wasserstoff oder einen geradkettigen oder verzweigten $C_1$-$C_5$-Alkylrest, Benzyl, 1-2fach mit $C_1$-$C_4$-Alkyl oder Halogen substituiertes Benzyl, Alkalimetall oder Ammonium ($NH_4^+$) oder mit $C_1$-$C_4$-Alkyl oder Hydroxy-$C_1$-$C_4$-alkyl substituiertes Ammoniumion.

Die Erfindung betrifft die reinen Enantiomeren mit der in der allgemeinen Formel I angegebenen absoluten Konfiguration 4R, 6S, wobei die offenkettigen Carbonsäuren bzw. Ester und Salze die absolute Konfiguration 3R, 5S besitzen.

Unter den Substituenten $R^1$ sind bevorzugt :

Cyclopentyl und Cyclohexyl.

Unter den Substituenten $R^2$, $R^3$ sind bevorzugt :

Wasserstoff, Halogen, Trifluormethyl, Alkyl oder Alkoxy mit je 1 bis 4 C-Atomen.

Unter den Substituenten $R^4$ sind bevorzugt :

Wasserstoff, Methyl, Äthyl, Isopropyl, Isobutyl, Benzyl, Natrium, Kalium, Ammonium, Trishydroxymethyl-methylamin.

Unter den Substituenten $R^1$ sind die im folgenden aufgeführten besonders bevorzugt :

Cyclopentyl und Cyclohexyl.

Unter den Substituenten $R^2$, $R^3$ sind besonders bevorzugt :

Wasserstoff, 2-Methyl, 2-Trifluormethyl, 2,4-Dimethyl, 2-Methyl-4-chlor, 2-Chlor-4-methyl, 2,4-Bistrifluormethyl, 2-Äthyl, 2-Isopropyl, 2-Isobutyl, 2-Chlor, 2-Fluor, 2-Brom, 2,4-Dichlor, 2,4-Difluor, 2-Methoxy, 4-Methoxy, 4-Dimethoxy.

Unter den Substituenten $R^4$ sind besonders bevorzugt :

Wasserstoff, Methyl, Äthyl, Benzyl, Natrium, Kalium, Ammonium, Trishydroxymethyl-methylamin.

Die Erfindung betrifft ferner ein Verfahren zur Herstellung von Verbindungen der Formeln I und Ia, das dadurch gekennzeichnet ist, daß man

a) die Phosphoniumsalze der Formel II

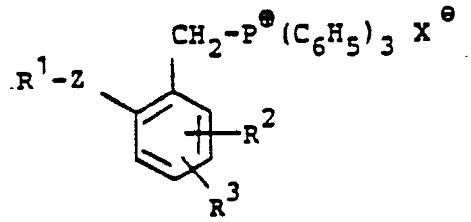

II

worin $R^1$, $R^2$, $R^3$ und Z die zur Formel I angegebene Bedeutung haben und X=Cl, Br, J ist, mit dem chiralen Aldehyd der Formel III umsetzt,

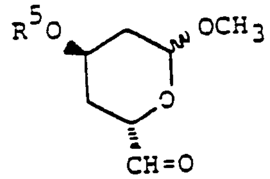

III

worin $R^5$ eine gegen Basen und schwache Säuren stabile Schutzgruppe bedeutet, z. B. die

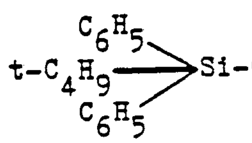

-Gruppe,

zu einer Verbindung der Formel IV

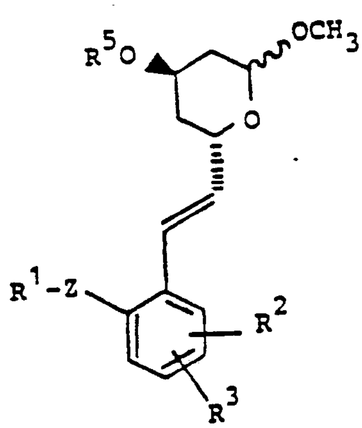

IV

worin $R^1$, $R^2$, $R^3$ und Z die zur Formel I, $R^5$ die zur Formel III gegebene Bedeutung haben (und AB die (—CH=CH—)-Gruppe darstellt),

b) in einer Verbindung der allg. Formel IV die Methylacetalfunktion sauer hydrolysiert zu einem Lactol der Formel V,

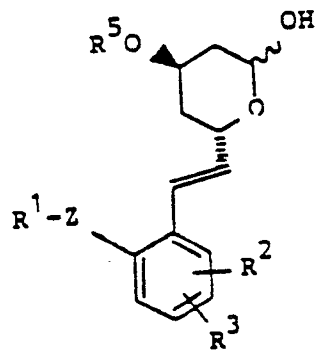

V

worin $R^1$, $R^2$, $R^3$ und Z die zur Formel I, $R^5$ die zur Formel III gegebene Bedeutung hat (und A-B die (—CH=CH—)-Gruppe darstellt),

c) die Verbindung der allg. Formel V oxydiert zu einem Lacton der Formel VI,

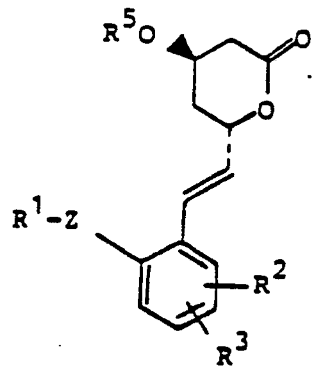

VI

worin $R^1$, $R^2$ und $R^3$ und Z die zur Formel I, $R^5$ die zur Formel III gegebene Bedeutung hat (und A-B die (—CH=CH—)-Gruppe darstellt),

d) in einer Verbindung der allg. Formel VI die Schutzgruppe $R^5$ abspaltet zu einer Verbindung der Formel I, worin $R^1$, $R^2$, $R^3$ und Z die zur Formel I angegebenen Bedeutungen haben und A-B die (—CH=CH—)-Gruppe darstellt, gegebenenfalls eine Verbindung der allgemeinen Formel I bei der A-B eine (—CH=CH—) Gruppe darstellt, hydriert zu einer Verbindung der allgemeinen Formel I, in der A-B eine ($—CH_2—CH_2—$)-Gruppe darstellt, wobei die Hydrierung auch bei den Verbindungen der Formeln IV, V oder VI erfolgen kann zu entsprechenden Verbindungen, worin A-B die ($—CH_2—CH_2—$)-Gruppe darstellt, gegebenenfalls ein Hydroxylacton I in die entsprechenden freien Hydroxysäuren Ia bzw. deren Salze überführt oder gegebenenfalls den entsprechenden Ester aus den freien Hydroxysäuren Ia bzw. aus dem Hydroxylacton I darstellt.

Die im erfindungsgemäßen Verfahren als Ausgangsmaterial verwandten Phosphoniumsalze der allgemeinen Formel II, wenn $Z=CH_2$ und $R^1$, $R^2$, $R^3$ die zur allg. Formel I gegebene Bedeutung haben, erhält man z. B. durch Umsetzung der entsprechenden substituierten Benzylhalogenide der allg. Formel XIII (vgl. Schema 1)

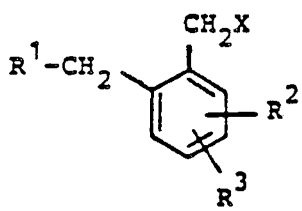

XIII

X=Cl, Br, J.

mit Triphenylphosphin in Acetonitril, Toluol oder anderen vergleichbaren Lösungsmitteln. Zur Herstellung der substituierten Benzylhalogenide XIII kann man, wenn $R^1$, $R^2$ und $R^3$ die zur allg. Formel I angegebene Bedeutung haben, analog dem Verfahren arbeiten, das von L. A. Walter et al., J. Heterocycl. Chem. 14, 47 (1977) beschrieben wurde (Schema 1-Weg A). In Abhängigkeit von $R^1$, ist es vorteilhaft, zur Herstellung der Verbindung der allg. Formel XIII (Schema 1) den Syntheseweg B einzuschlagen (vgl. auch Bsp. 1, 4, 5, 6).

Schema 1

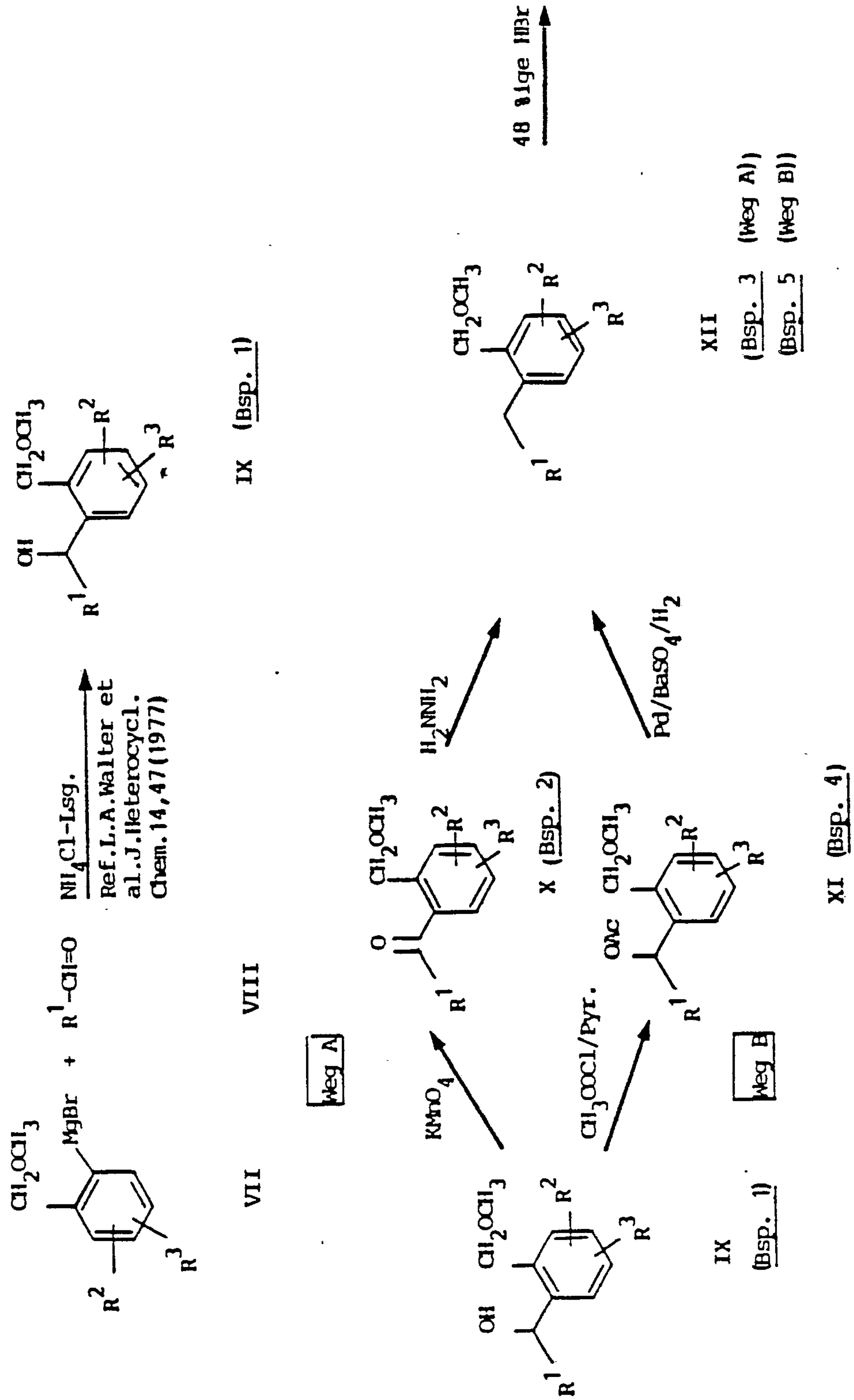


VII

VIII

IX (Bsp. 1)

X (Bsp. 2)

XI (Bsp. 4)

XII (Bsp. 3 (Weg A)) (Bsp. 5 (Weg B))

Fortsetzung Schema 1

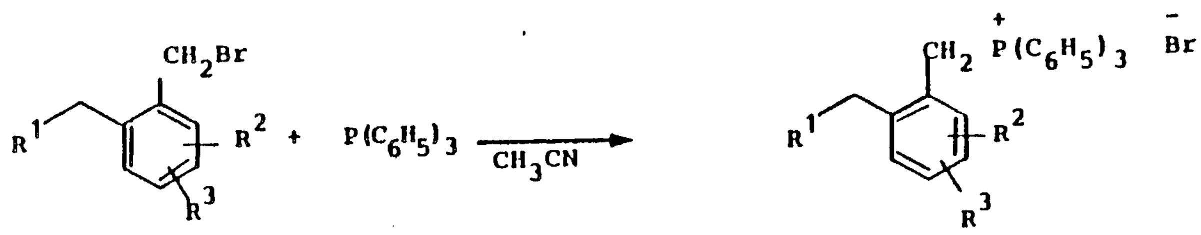


XIII (Bsp. 6)

II ($Z=CH_2$) (Bsp. 7)

Die im erfindungsgemäßen Verfahren als Ausgangsmaterial verwandten Phosphoniumsalze der allg. Formel II, wenn $Z=CH_2—CH_2$ ist und $R^1$, $R^2$ und $R^3$, die zur allg. Formel I genannte Bedeutung haben, erhält man, wie in Schema 2 dargestellt (vgl. auch Beispiele 8 bis 11).

Schema 2

$$R^1-CH_2-X \quad + \quad (C_6H_5)_3P \longrightarrow R^1-CH_2-\overset{\oplus}{P}(C_6H_5)_3 \quad X^{\ominus}$$

(XIV) (XV)

X=Br, Cl (Bsp. 8)

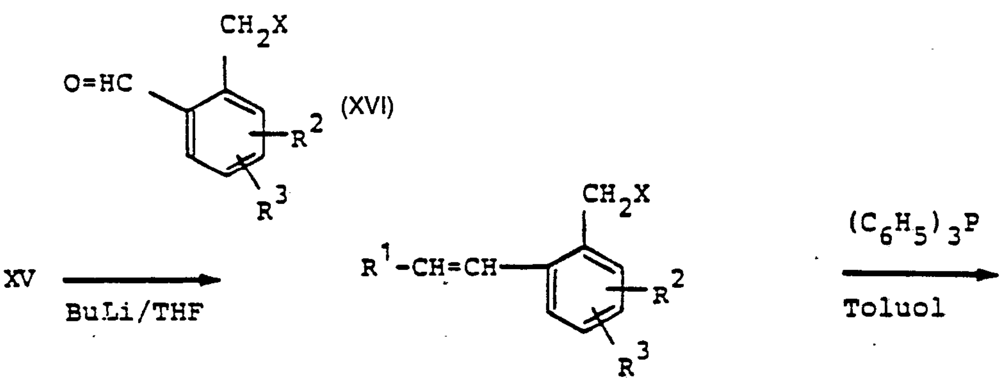


(XVII) (Bsp. 9)

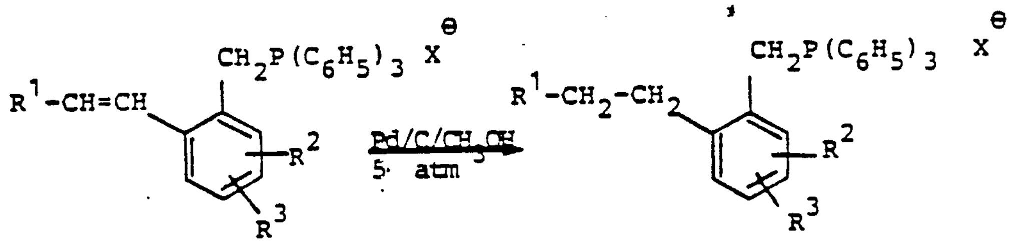


(XVIII) (Bsp. 10) (II) (Bsp. 11)

$(Z=-CH_2-CH_2-)$

Den im erfindungsgemäßen Verfahren als Ausgangsmaterial verwandten chiralen Aldehyd der Formel III erhält man nach einem literaturbekannten Verfahren (Yuh Lin, J. R. Falck, Tetrahedron Letters 23, 4305-4308 (1982) aus dem entsprechenden Alkohol durch Oxidation mit z. B. $CrO_3$.

Die Umsetzung des chiralen Aldehyds der Formel III mit einem Phosphoniumsalz der Formel II nach Wittig (z. B. Wittig, Haag, Chem. Ber. 88, 1654 (1955)) ergibt die Verbindungen der Formel IV, wobei eine bevorzugte Ausführungsform darin besteht, daß man die Phosphoniumsalze der Formel II in einem Lösungsmittel wie Tetrahydrofuran, Dimethylsulfoxid, DME, löst bzw. suspendiert und mit einer geeigneten starken Base wie z. B. Natriumhydrid, Kaliumtert. butylat, Li-äthylat oder Butyllithium die entsprechenden Phosphorane freisetzt und anschließend den Aldehyd der Formel III zugibt und bei — 10 °C bis + 50 °C 1-6 Std. reagieren läßt.

Die Verbindungen der Formel IV werden dabei überwiegend in Form der Mischung der E/Z-Olefine erhalten. Mischungen von E/Z-Olefinen können gegebenenfalls chromatographisch aufgetrennt werden. Die reinen Z-Olefine können auch, wie bei (G. Drefahl Chem. Ber. 94, 907 (1961) beschreiben durch Belichten der E/Z-Mischung in Lösungen, wie z. B. Toluol, Nitrobenzol, erhalten werden.

Die entsprechenden reinen E-Olefine können wie von De Tar et. al. in J. Amer. Chem. Soc. 78, 474 (1955) beschrieben durch Erwärmen der E/Z-Mischungen in Lösung in Gegenwart von Jod erhalten werden.

In den Verbindungen der Formel IV läßt sich die Methylacetalschutzgruppe in der allgemein üblichen Weise durch saure Hydrolyse selektiv abspalten, bevorzugt mit einer Mischung von Eisessig, Tetrahydrofuran, Wasser im Verhältnis 3 : 2 : 2 bei + 20 bis + 90 °C innerhalb 6-24 Stunden.

Die Oxydation der Verbindungen der Formel V zu einem Lakton der Formel VI kann durch Oxydationsmittel wie $CrO_3 \times 2Pyr$, Pyridiumchlorochromat in inerten Lösungsmitteln, wie z. B. Methylenchlorid oder Chloroform erfolgen. Eine weitere Möglichkeit der Oxydation besteht in der Reaktion mit Thioanisol /$Cl_2$/$NEt_3$ in Tetrachlorkohlenstoff oder in der Umsetzung mit DMSO/Oxalylchlorid/$NEt_3$ bei — 20 °C.

Zur Darstellung der Verbindungen der Formel I, wird die Schutzgruppe $R^5$ in den Verbindungen der Formel VI abgespaltet. Dies kann mit starken Säuren wie 5 normaler Salzsäure oder Schwefelsäure bei — 10 °C bei + 30 °C geschehen, oder mit Fluoridionen, bevorzugt durch lösen der Verbindungen der Formel VI in Tetrahydrofuran oder Diäthyläther und Zugabe einer Mischung von Tetrabutylammoniumfluorid und Eisessig mit anschließendem Rühren bei 0 °C bis 40 °C zwischen 1 bis 12 Stunden.

Verbindungen der Formel I, bei denen A-B eine (CH=CH) Gruppe darstellt, werden nach allgemein üblicher Methode, zweckmäßig bei Temp. zwischen 20° und 40° mit Wasserstoff in Gegenwart eines Metall-Katalysators, bevorzugt Palladium, Platin, $PtO_2$ oder $PdO_2$ zu Verbindungen der Formel I, bei denen A-B eine $—CH_2—CH_2$-Gruppe bedeutet, hydriert. Dabei kann bei Normaldruck in üblichen Lösungsmitteln wie Tetrahydrofuran, Essigester, Methanol, niedermolekularen Alkohlen, Eisessig, Chloroform hydriert werden, oder in Autoklaven bei erhöhtem Druck 2-50 atm. Die Hydrierung der —CH=CH-Gruppe kann auch bei den Verbindungen der Formeln IV, V oder VI erfolgen.

Die resultierenden Verbindungen der Formel I können in einfacher Weise durch Eindampfen des Lösungsmittels isoliert werden, gegebenenfalls nach chromatographischer Reinigung.

Die Verbindungen der Formel I fallen in optisch reiner Form an. Bezüglich der Konfiguration der Doppelbindung (A-B)=—CH=CH— erhält man E/Z-Gemische, diese lassen sich auf allen Synthesestufen chromatographisch auftrennen, oder zur E-Form isomerisieren. (vgl. dazu De Tar et. al J. Amer. Chem. Soc. 78, 475 (1955)).

Verbindungen der Formel I, in Form des δ-Lactons können in alkalischem Medium zu den entsprechenden Salzen der Dihydroxysäuren verseift werden, z. B. mit NaOH oder KOH in einem niedermolikularen Alkohol wie Methanol oder in Ethern wie Dimethoxyethan oder THF, gegebenenfalls in Gegenwart von Wasser. In den erhaltenen Salzen der Dihydroxysäuren läßt sich das Alkalikation nach Ansäuern in Ionenaustauschern in üblicher Weise gegen beliebige Kationen austauschen. Dazu läßt man z. B. die Dihydroxysäuren durch eine mit einem Kationenaustauscher, wie z. B. auf Basis Polystyrol/Divinylbenzol (®) Amberlite CG-150 oder ®) Dowex-CCR-2) gefüllte Säule laufen. Der Kationenaustauscher ist mit dem gewünschten Kation beladen, z. B. mit Ammoniumionen, die sich von einem primären, sekundären oder tertiären Amin ableiten. Das gewünschte Salz erhält man durch Eindampfen des Eluats.

Ammoniumsalze der Dihydroxysäuren die sich von einem primären, sekundären oder tertiären Amin ableiten, kann man auch herstellen, indem man die freien Dihydroxysäuren in einer alkoholischen Lösung mit einer äquimolaren Menge des entsprechenden Amins versetzt und das Lösungsmittel eindampft.

Die freien Dihydroxysäuren Ia der δ-Lactone I lassen sich nach üblichen Methoden z. B. mit einem Diazoalkan verestern. So kann man z. B. Verbindungen der Formel I mit $R^1$=H bei Temperaturen zwischen — 40 und + 20 °C mit einem Diazoalkan verestern, wobei die üblichen Lösungsmittel wie z. B. Diethylether, Tetrahydrofuran, Chloroform oder niedermolekulare Alkohole wie Methanol verwendet werden können. Die resultierenden Ester können in einfacher Weise durch Eindampfen des Lösungsmittels isoliert und ggf. chromatographisch gereinigt werden. Eine weitere Veresterungsmethode besteht darin, daß man Salze der Dihydroxysäuren Ia in Gegenwart einer Base wie z. B. eines Metallalkoholates oder Metallcarbonates in einem geeigneten Lösungsmittel mit einem Alkylierungsmittel umsetzt. Als Metallalkoholate kommen z. B. Natriummethylat, Natriumethylat oder Kaliumtertiärbutylat in Betracht. Als geeignetes Lösungsmittel kommen Alkohole wie z. B. Methanol oder tert. Butanol, Ether wie Tetrahydrofuran oder 1,2-Dimethoxyethan und insbesondere dipolare approtische Lösungsmittel wie Dimethylformamid, Dimethylsulfoxid, Acetonitril, oder N-Methylpyrrolidon in Betracht. Zur Darstellung von Estern der Dihydroxysäuren eignet sich auch die Methode der Umesterung mit Überschuß an Alkoholen wie z. B. Methanol, Ethanol, Isopropanol.

Sofern die einzelnen Reaktionsprodukte nicht bereits in genügend reiner Form anfallen, so daß sie für den folgenden Reaktionsschritt eingesetzt werden können, empfiehlt sich eine Reinigung mittels Kristallisation, Säulen-, Dünnschicht- oder Hochdruckflüssigkeitschromatografie.

Falls das Aldehyd der Formel III nicht als reines Enantiomeres vorliegt, können auch Mischungen der enantiomeren Endprodukte entstehen, die nach allgemein üblichen Verfahren aufgetrennt werden können.

Außer den in den Beispielen beschriebenen Verbindungen lassen sich nach den erfindungsgemäßen Verfahren die folgenden Verbindungen herstellen :

(+)-E-6S-[2-(2-(2-Cyclohexyl)ethyl)-6-chlorphenylethenyl]-4R-hydroxy-3,4,5,6-tetrahydro-2H-pyran-2-on

(+)-E-6S-[2-(2-(2-(Cyclohexylmethyl)-6-chlorphenylethenyl]-4R-hydroxy-3,4,5,6-tetrahydro-2H-pyran-2-on

(+)-E-6S-[2-(2-(2-(Cyclohexyl)methyl)-6-chlorphenyl-ethyl]-4R-hydroxy-3,4,5,6-tetrahydro-2H-pyran-2-on

(+)-E-6S-[2-(2-(2-(Cyclohexylmethyl)-4,6-dimethylphenylethenyl]-4R-hydroxy-3,4,5,6-tetrahydro-2H-pyran-2-on

(+)-E-6S-[2-(2-(2-(Cyclohexylmethyl)-4-methyl-6-chlorphenylethenyl]-4R-hydroxy-3,4,5,6-tetrahydro-2H-pyran-2-on

(+)-E-6S-[2-(2-(2-(Cyclohexylmethyl)-4-chlor-6-methylphenyl-ethenyl]-4R-hydroxy-3,4,5,6-tetrahydro-2H-pyran-2-on

(+)-E-6S-[2-(2-(2-(4,4-Dimethylcyclohexylmethyl)-4,6-dimethylphenyl-ethenyl]-4R-hydroxy-3,4,5,6-tetrahydro-2H-pyran-2-on

(+)-E-6S-[2-(2-(2-(4,4-Dimethylcyclohexylmethyl)-4-methyl-6-chlor-phenyl-ethenyl]-4R-hydroxy-3,4,5,6-tetrahydro-2H-pyran-2-on

(+)-E-6S-[2-(2-(2-(4,4-Dimethylcyclohexylmethyl)-4-chlor-6-methyl-phenyl-ethenyl]-4R-hydroxy-3,4,5,6-tetrahydro-2H-pyran-2-on

## Biologische Testsysteme :

1. HMG-CoA-Reduktase-Aktivität in Enzympräparationen.

Die HMG-CoA-Reduktase-Aktivität wurde an solubilisierten Enzympräparationen aus Lebermikrosomen von Ratten gemessen, die nach Umstellung im Tag-Nacht-Rhythmus mit Cholestyramin (®Cuemid) induziert wurden. Als Substrat diente (S,R) $^{14}C$-HMG-CoA, die Konzentration von NADPH wurde während der Inkubation durch ein regenerierendes System aufrechterhalten. Die Abtrennung von $^{14}C$-Mevalonat vom Substrat und anderen Produkten (z. B. $^{14}C$-HMG) erfolgte über Säulenelution, wobei das Elutionsprofil jeder Einzelprobe ermittelt wurde. Auf die ständige Mitführung von $^{3}H$-Mevalonat wurde verzichtet, weil es bei der Bestimmung um die Relativangabe der Hemmwirkung handelt. In einer Versuchsreihe wurden jeweils die enzymfreie Kontrolle, der enzymhaltige Normalansatz (= 100 %) und solche mit Präparatezusätzen, Endkonzentration $10^{-5}$ bis $10^{-9}$ M, zusammen behandelt. Jeder Einzelwert wurde als Mittelwert aus 3 Parallelproben gebildet. Die Signifikanz der Mittelwertsunterschiede zwischen, praparatefreien und präparatehaltigen Proben wurde nach dem t-Test beurteilt.

Nach der oben beschriebenen Methode wurden von den erfindungsgemäßen Verbindungen z. B. folgende Hemmwerte auf die HMG-CoA-Reduktase ermittelt :

(Siehe Tabelle Seite 10 f.)

| Beispiel (Tab. 8) | $R^1$ | $R^2$ | $R^3$ | Z | $IC_{50}$-Wert [M] |
|---|---|---|---|---|---|
| 16 b | 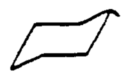 | H | H | $CH_2$ | $9.0 \times 10^{-8}$ |
| 16 f | 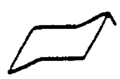 | o-Cl | p-Cl | $CH_2$ | $1,9 \times 10^{-8}$ |
| 16 c | 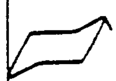 | o-$CH_3$ | H | $CH_2CH_2$ | $6,5 \times 10^{-7}$ |
| 16 w | 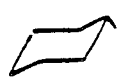 | o-Cl | H | $CH_2$ | $7,1 \times 10^{-8}$ |

2. Supprimierung bzw. Inhibierung der HMG-CoA-Reduktase in Zellkulturen von Fibroblasten.

Monolayers von Fibroblasten (L-Zellen) in lipoproteinfreiem Nährmedium wurden mit entsprechenden Konzentrationen der Prüfsubstanzen eine bestimmte Zeit (z. B. 3 Stunden) inkubiert, dann wurde die HMG-CoA-Reduktaseaktivität der Zellen, modifiziert nach Chang et al. (J. Biol. Chem. 256, 6174 (1981)) bestimmt. Dazu wurden die Zellextrakte mit D,L[$^3$H]-HMGCoA inkubiert und das unter der Einwirkung der vorhandenen HMG-CoA-Reduktaseaktivität aus den Zellen gebildete Produkt [$^3$H]-Mevalonat nach Cyclisierung zu [$^3$H]-Mevalonolacton dünnschichtchromatgraphisch vom Ausgangsprodukt getrennt und unter Verwendung eines internen Standards von [$^{14}$C]-Mevalonat die Menge des in der Zeiteinheit gebildeten [$^3$H]-Movalonats, bezogen auf Zellproteinmenge bestimmt. Die unter Zusatz einer bestimmten Konzentration eines Prüfpräparates gefundene HMG-CoA-Reduktaseaktivität der Zellkultur wurde prozentual auf jene einer gleichbehandelten ohne Prüfpräparat, jedoch gleicher Lösungsmittelkonzentration, bezogen.

(Siehe Tabelle Seite 11 f.)

3. Prüfung von Substanzen auf Hemmung der HMG-CoA-Reduktase in Zellkulturen

konfluente Zellkultur (Monolayer) von HEP-G2-Zellen

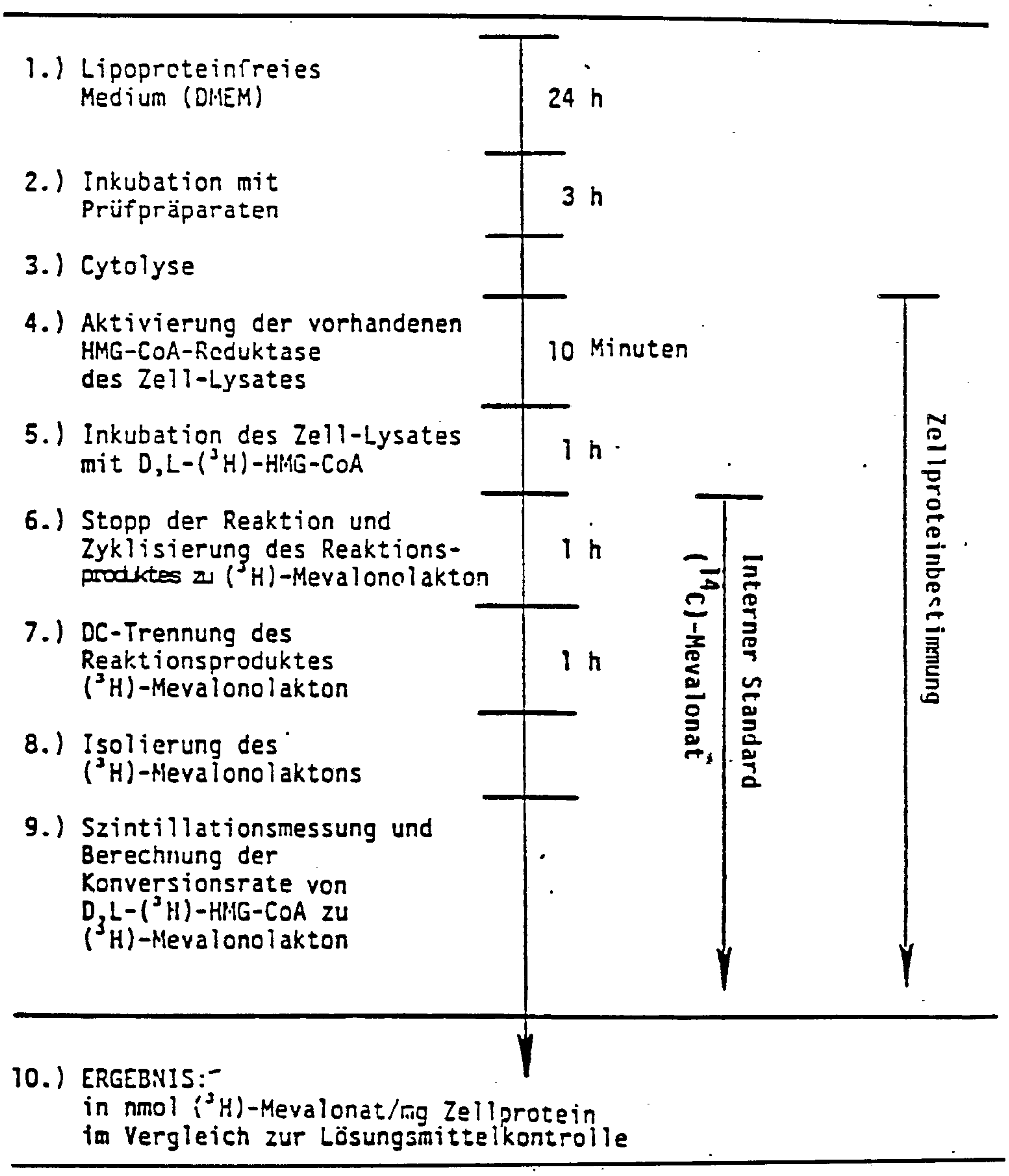


Nach der oben beschriebenen Methode wurden von den erfindungsgemäßen Verbindungen z. B. folgende Hemmwerte auf die HMG-CoA-Reduktase (in HEP-G2-Zellen) ermittelt (Der $IC_{50}$-Wert (M) ist diejenige Konzentration der Verbindung, die eine 50 %ige Hemmung der HMG-CoA-Reduktase-Aktivität bewirkt) :

(Siehe Tabelle Seite 12 f.)

| Beispiel (Tab. 8) | $R^1$ | $R^2$ | $R^3$ | Z | $IC_{50}$-Wert(M) |
|---|---|---|---|---|---|
| 16 b | 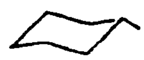 | H | H | $CH_2$ | $3.8 \times 10^{-4}$ |
| 16 f | 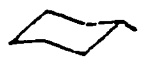 | o-Cl | p-Cl | $CH_2$ | $1.9 \times 10^{-6}$ |
| 16 c | 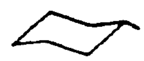 | $o-CH_3$ | H | $CH_2CH_2$ | $2.0 \times 10^{-4}$ |
| 16 h | 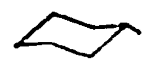 | o-Cl | H | $CH_2$ | $3.2 \times 10^{-6}$ |
| Mevinolin | | | | | $6.0 \times 10^{-6}$ |

Die Verbindungen der allg. Formel I bzw. Ia zeichnen sich aus durch starke Hemmung der HMG-CoA-Reduktase, des geschwindigkeitsbestimmenden Enzyms der Cholesterinbiosynthese. Das Enzym HMG-CoA-Reduktase ist in der Natur weit verbreitet. Es katalysiert die Bildung von Mevalonsäure aus HMG-CoA. Diese Reaktion ist ein zentraler Schritt der Cholesterinbiosynthese (vgl. J. R. Sabine in CRC Series in Enzyme Biology : 3-Hydroxy-3-methylgluraryl Coenzym A Reduktase, CRC Press Inc. Boca Raten, Florida 1983 (ISBN 0-8493-6551-1)).

Hohe Cholesterinspiegel werden mit einer Reihe von Erkrankungen, wie z. B. koronarer Herzkrankheit oder Arteriosklerose in Verbindung gebracht. Daher ist die Senkung erhöhter Cholesterinspiegel zur Vorbeugung und Behandlung solcher Erkrankungen ein therapeutisches Ziel.

Ein Ansatzpunkt dazu liegt in der Hemmung bzw. Verminderung der endogenen Cholesterinbiosynthese. Hemmstoffe der HMG-CoA-Reduktase blockieren die Cholesterinbiosynthese auf einer frühen Stufe.

Die Verbindungen der allg. Formel I bzw. Ia eignen sich daher als Hypolipidemika und zur Behandlung bzw. Prophylaxe arteriosklerotischer Veränderungen.

Die Erfindung betrifft daher auch pharmazeutische Präparate auf Basis dieser Verbindungen sowie ihre Verwendung als Arzneimittel, insbesondere als Hypolipidemika und zur Prophylaxe arteriosklerotischer Veränderungen.

Die Anwendung der Verbindungen der Formel I bzw. Ia als Hypolipidemika oder Anti-arteriosklerotika erfolgt in oralen Dosen von 3 bis 2 500 mg, vorzugsweise jedoch im Dosisbereich von 10-500 mg. Diese Tagesdosen können nach Bedarf auch in zwei bis vier Einzeldosen aufgeteilt werden oder in Retardform verarbeitet werden. Das Dosierungsschema kann vom Typ, Alter, Gewicht, Geschlecht und medizinischen Zustand des Patienten abhängen.

Ein zusätzlicher cholesterinsenkender Effekt läßt sich durch gleichzeitige Gabe der erfindungsgemäßen Verbindungen mit Gallensäurebindenden Stoffen, wie z. B. Anionenaustauscherharzen, erzielen. Die erhöhte Gallensäureausscheidung führt zu einer verstärkten Neusynthese und damit zu einem erhöhten Cholersterinabbau (vgl. M. S. Brown, P. T. Koranen u. J. C. Goldstein Science 212, 628 (1981) ; M. S. Brown, J. C. Goldstein Spektrum der Wissenschaft 1985, 1, 96).

Die erfindungsgemäßen Verbindungen der Formel I bzw. Ia können in Form der δ-Lactone, als freie Säuren oder in Form ihrer physiologisch unbedenklichen anorganischen oder organischen Salze oder als Ester zur Anwendung kommen. Säuren und Salze bzw. Ester können in Form ihrer wässrigen Lösungen oder Suspensionen oder auch gelöst oder suspendiert in pharmakologisch unbedenklichen organischen Lösungsmitteln wie ein- oder mehrwertigen Alkoholen wie z. B. Ethanol, Ethylenglykol oder Glycerin, in Triacetin, in Alkohol-Acetaldehyddiacetalgemischen, Ölen wie z. B. Sonnenblumenöl oder Lebertran, Ethern wie z. B. Diethylenglykoldimethylether oder auch Polyethern wie z. B. Polyethylenglykol oder auch in Gegenwart anderer pharmakologisch unbedenklicher Polymerträger wie z. B. Polyvinylpyrrolidon oder in festen Zubereitungen zur Anwendung gelangen.

Für die Verbindungen der Formel I bzw. I a sind feste, oral verabreichbare Zubereitungsformen bevorzugt, die die üblichen Hilfsstoffe enthalten können. Sie werden nach üblichen Methoden hergestellt.

Als Zubereitungen für die orale Anwendung eignen sich insbesondere Tabletten, Dragees oder Kapseln. Eine Dosierungseinheit enthält vorzugsweise 10 bis 500 mg Wirkstoff.

Die Verbindungen der Formel II, III, IV, V und VI sind neu und stellen wertvolle Zwischenprodukte für die Herstellung von Verbindungen der Formel I dar. Die Erfindung betrifft daher auch diese Verbindungen sowie Verfahren zu ihrer Herstellung.

Herstellung der Ausgangsverbindungen II (Schema 1, z = $-CH_2-$)

Beispiel 1

Allgemeine Vorschrift zur Herstellung der Verbindungen der allg. Formel IX (vgl. L. A. Walter et al., J. Heterocycl. Chem. 14, 47 (1977)).

Beispiel 1a

($R^2$, $R^3$ = H, $R^1$ = c-$C_6H_{11}$)
Cyclohexyl-2-(methoxymethyl)phenyl-methanol IX

6,34 (0,26 Mol) Magnesiumspäne, die mit etherischer Salzsäure angeätzt und danach gut getrocknet wurden, wurden mit einem Kristall Jod versetzt und mit 60 ml abs. THF überschichtet.

50 g (0,25 Mol) 2-Methoxymethyl-brombenzol (2-Brombenzylmethylether VII) in 500 ml abs. THF wurden langsam zugetropft. Nach Beendigung der Grignardreaktion wurden bei 50°, 28,1 g (0,25 Mol) Cyclohexylaldehyd VIII in 200 ml abs. THF langsam zugetropft. Anschließend wurde 2 Stunden am Rückfluß erhitzt. Dann wurde das Tetrahydrofuran i. Vak. abdestilliert. Die org. Phasen wurden vereint, getrocknet mit $MgSO_4$, filtriert und i. Vak. eingeengt.

Ausbeute : 49 g 84 % d. Th. IX
Rf = 0,25 Laufmittel : Cyclohexan/Essigester 9 : 1

Beispiel 2

Vorschrift zur Herstellung der Verbindungen der allg. Formel X-Weg A

Beispiel 2a

($R^2$, $R^3$ = H, $R^1$ = Cyclohexyl)
Cyclohexyl-2-(methoxymethyl)phenyl-keton Xa

47 g (0,2 Mol) cyclohexyl-2-(methoxymethyl) phenyl-methanol (Beispiel 1a) wurden in 380 ml Dioxan, 572 ml $H_2O$ und Zusatz von 5,72 g KOH gelöst und auf 40 °C erwärmt. Zu dieser Mischung wurde unter Rühren 24,94 g (0,157 Mol) Kaliumpermanganat in kleineren Portionen bis zur jeweiligen Entfärbung zugegeben. Nach beendeter Zugabe wurde 45' bei 90 °C weitergerührt. Danach wurde abgekühlt und mit 4 × 200 ml Äther extrahiert. Die organischen Phasen wurden vereinigt, mit $H_2O$ gewaschen und mit $MgSO_4$ getrocknet, eingeengt i. Vak., anschließend wurde über Kieselgel mit Cyclohexan, Essigester = 1 : 1 filtriert.

Ausbeute : 40 g 68 % d. Th. Xa
Rf = 0.42 Laufmittel : Cyclohexan/Essigester 1 : 1

Beispiel 3

Vorschrift zur Herstellung der Verbindungen der allg. Formel XIII-Weg A

Beispiel 3a ($R^2$, $R^3$ = H, $R^1$ = Cyclohexyl)

2-Methoxymethyl-(cyclohexyl-methyl)-benzol XIIa

34,5 g (0.146 Mol) Cyclohexyl-2-(methoxymethyl)phenyl-keton (Beispiel 2a) werden in 232 ml Triethylenglykol zusammen mit 41.1 g KOH gelöst. Dann gibt man 24.68 ml 91 %iges Hydrazin zu und erwärmt unter Rühren 1 Stunde auf 100 °C und 5 Stunden auf 180 °C. Dann läßt man abkühlen, fügt 400 ml Eiswasser zu, und extrahiert 4 × mit 250 ml Äther. Die vereinigten Ätherphasen werden mit ges. NaCl-Lösung gewaschen, getrocknet und i. Vak. eingeengt. Über $SiO_2$ wird mit Cyclohexan/Essigester 9 : 1 filtriert.

Ausbeute : 22.9 g (72 % d. Th.) XIIa
Rf = 0.48

Laufmittel : Chloroform

Beispiel 4

Vorschrift zur Herstellung der Verbindungen der allg. Formel XI-Weg B

Beispiel 4a ($R^1$ = Cyclohexyl, $R^2$, $R^3$ = H)

(2-Methoxymethyl)phenyl-cyclohexyl-acetyloxy methan XIa

16,8 g (0.072 Mol) (2-Methoxymethyl)phenyl-cyclohexyl-methanol (Beispiel 1a) wurden unter Stickstoff bei 0-5 °C in 230 ml $CH_2Cl_2$ abs. gelöst, vorgelegt und mit 34.9 g (0.43 Mol) abs. Pyridin versetzt. Unter Eiskühlung wurden anschließend während 3/4 Stdn. 10.17 ml (0.144 Mol) Acetylchlorid unter Kühlung zugetropft. 1/2 Stunde wurde nachgerührt.

Dann wurde die Reaktionsmischung auf Eis gegeben, und mit $CH_2Cl_2$ extrahiert. Die org. Phase wurde eingeengt. Der Rückstand wurde 3 × mit Toluol versetzt und Reste von Pyridin wurden azeotrop abdestilliert ; anschließend wurde in H. V getrocknet.

Ausbeute : 19.1 g (96 % d. Th.) XIa
Rf = 0.19
Laufmittel : Chloroform

Beispiel 5

Vorschrift zur Herstellung der Verbindungen der allgemeinen Formel XII-Weg B

Beispiel 5a ($R^1$ = Cyclohexyl, $R^2$, $R^3$ = H)

2-Methoxymethyl-(cyclohexyl)-benzol XIIa

6 g Palladium-Bariumsulfat wurden in 200 ml MeOH abs. vorhydriert. Dann wurden 19.9 g (0.072 Mol) (2-Methoxymethyl) phenyl-cyclohexyl-acetyloxy-methan (Beispiel 4a) + 5.92 g (0.072 Mol) Natriumacetat zugegeben und hydriert. Nach 2 Stunden war die $H_2$-Aufnahme beendet. Über ein Klärschichtfilter mit Kieselgel wurde die Aktivkohle unter $N_2$ abgesaugt.

Das Filtrat wurde im Vak. eingeengt und der Rückstand über Kieselgel mit Cyclohexan/Essigester 9 : 1 filtriert.

Ausbeute : 8.8 g 56 % d. Th. XIIa
Rf = 0.48
Laufmittel : Chloroform

Beispiel 6

Vorschrift zur Herstellung der Verbindungen der allg. Formel XIII

Beispiel 6a 2-Cyclohexyl-methyl-benzylbromid XIIIa ($R^1$ = Cyclohexyl, $R^2$, $R^3$ = H)

12.9 g (0.059 Mol) 2-Methoxymethyl-(cyclohexylmethyl)-benzol (Bsp. 3a) wurden in 130 ml 48 %iger wässeriger Bromwasserstoffsäure gelöst und 3 Stdn. unter Rückfluß gerührt.

Nach dem Abkühlen wurden 200 ml Toluol zugefügt und das Gemisch wurde kräftig durchgerührt. Die Toluolphase wurde abgetrennt, die wässerige Phase 2 × mit Toluol extrahiert. Die vereinigten organischen Phasen wurden 1 × mit Eiswasser, 1 × mit gesättigter $NaHCO_3$-Lösung und 1 × mit gesättigter NaCl-Lösung gewaschen, dann wurde mit $MgSO_4$ getrocknet und i. Vak. eingeengt. Der Rückstand wurde über Kieselgel mit Cyclohexyn/Essigester 9 : 1 filtriert.

Ausbeute : 14.6 g (93 % d. Th.) XIIIa
Rf = 0.8
Laufmittel : Cyclohexan/Essigester 4 : 1

Beispiel 7

Vorschrift zur Herstellung der Verbindungen der allg. Formel II (Z = $CH_2$)

Beispiel 7a 2-Cyclohexylmethyl-benzyltriphenylphosphoniumbromid IIa
($R^1$ = Cyclohexyl, Z = $CH_2$, $R^2$, $R^3$ = H)

14.9 g (0.056 Mol) 2-Cyclohexylmethylbenzylbromid (Bsp. 6a) werden in 150 ml Toluol abs. gelöst, mit 16.18 g (0.062 Mol) Triphenylphosphin versetzt und 3 Stunden am Rückfluß erhitzt. Nach dem Abkühlen

wird der gebildete Kristallbrei über eine Nutsche abgesaugt. Der Filterrückstand wird 3 × mit Toluol und 2 × mit Diisopropyläther nachgewaschen, dann werden die Kristalle im Vakuumtrockenschrank bei 100 °C getrocknet.

Ausbeute : 28.1 g (95 % d. Th.) IIa

$C_{32}H_{34}PBr\ Mg$ : 529.48

Fp. 196-98 °C.

Beispiel 7b

Die Verbindung IIb (Z = $CH_2$) wurde in analoger Weise wie in Beispiel 7a beschreiben hergestellt (vgl. Tab. 1)

Tabelle 1

$R^3$ = H

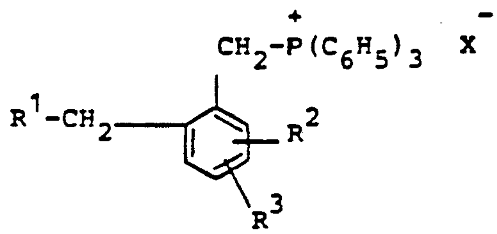

II (Z = $CH_2$

| $R^1$ | $R^2$ | X | Beispiel 7 | Schmp. °C | Ausbeute % |
|---|---|---|---|---|---|
| 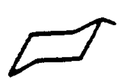 | o-Cl | Cl | b | 205 | 70 |

Herstellung der Ausgangsverbindungen II (Schema 2, Z = —$CH_2$—$CH_2$—)

Beispiel 8

Arbeitsvorschrift zur Darstellung der Phosphoniumsalze der allg. Formel XV

Beispiel 8a Cyclohexylmethyl-triphenyl-phophoniumbromid ($R^1$ = Cyclohexyl, X = Br) XVa

92.0 g (0.52 Mol) 2-Cyclohexylmethylbromid (XIV, $R^1$ = Cyclohexyl, X = Br) 136.7 g (0.52 Mol) Triphenylphosphin werden in 400 ml Acetonitril 6 Stunden am Rückfluß gekocht. Nach dem Abkühlen wird auf das halbe Volumen im Vak. eingeengt, im Eisbad abgekühlt und die ausgefallenen Kristalle werden abgesaugt und mit kaltem Acetonitril und Diäthyläther nachgewaschen. Die Kristalle werden i. Vak. bei 100 °C sorgfältig getrocknet.

Ausbeute : 84.1 g weiße Kristalle (45 % d. Th.) XVa

Fp. 216-217 °C.

Beispiel 9

Arbeitsvorschrift zur Darstellung der substituierten Benzylhalogenide der allg. Formel XVII (Tab. 2)

Beispiel 9a 2(2-(Cyclohexyl)-ethenyl)-benzylchlorid ($R^1$ = Cyclohexyl, X = Cl) XVIIa

57,5 g (0.16 Mol) Cyclohexylmethyl-triphenylphosphoniumbromid (Beispiel 8a) werden in abs. Tetrahydrofuran (300 ml) unter $N_2$ bei 0 °C aufgeschlämmt. Bei dieser Temperatur werden 101 ml (0.16 Mol) BuLi in Hexan zugetropft. Dabei bildet sich ein tiefrotes Phosphorylid. Man rührt noch ca. 15 Min., und tropft dann 27.21 g (0.16 + 0.016 Mol) 2-Chlormethylbenzaldehyd XVI (hergestellt nach G. Dreyfahl u. G. Plötner Chem. Ber. 94,907 (1961)) in 20 ml abs. THF gelöst innerhalb 30 Min. zu. Man rührt noch ca. 1 Std. nach, entfernt das Tetrahydrofuran im Vakuum, extrahiert den Rückstand mehrmals mit

einer Mischung 500 ml Pentan und 300 ml $H_2O$. Die vereinigten Pentanextrakte werden mit $MgSO_4$ getrocknet und eingeengt. Der Rückstand wird unter Zusatz von Aktivkohle mit Äther (300 ml) versetzt und erneut extrahiert. Der Ätherextrakt wird abgetrennt und über Nacht im Kühlschrank aufbewahrt. Der Äther wird abdekantiert vom ungelösten, filtriert und im Vak. eingeengt. Zurück bleibt ein hellgelbes Öl.

Ausbeute : 21.3 g (57 % d. Th.) XVIIa

Rf-Wert : 0.51 (E/Z-Gemisch) in Cyclohexan : Essigester = 4 : 1

Beispiel 9b

Nach der in Beispiel 9 angegebenen Vorschrift wurde das in Tab. 2 aufgeführte substituierte Benzylhalogenid der allg. Formel XVIIb hergestellt.

Tabelle 2

$R^3$ = H

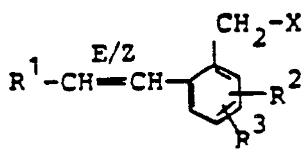

(XVII)

| $R^1$ | $R^2$ | X | Beispiel 9 | Schmp. °C $R_f$-wert CH/EE=4:1 | Ausbeute % |
|---|---|---|---|---|---|
| 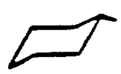 | o-Cl | Cl | b | 0.91 | 42 |

Beispiel 10

Arbeitsvorschrift zur Darstellung der Phosphoniumsalze der allgemeinen Formel XVIII (Tab. 3)

Beispiel 10a 2(2-(4-Cyclohexyl)-ethenyl)-benzyl-triphenyl-phosphoniumchlorid ($R^1$ = Cyclohexyl, X = Cl) XVIIIa

35.1 g (0.15 Mol) 2(2-(Cyclohexyl)-ethenyl)-benzylchlorid (Beispiel 9a) wurden zusammen mit 39.3 g (0.15 Mol) Triphenylphosphin in 220 ml Xylol gelöst und unter Rückfluß für ca. 3-6 Stunden erwärmt. Nach ca. 1/2 Stunde begann das gebildete unlöstliche Phosphoniumsalz auszufallen. Der Reaktionsverlauf wurde durch Dünnschichtchromatographie in $CH_3OH/CH_2Cl_2$ = 1/1 auf Kieselgelplatten verfolgt. Nach Beendigung der Reaktion wurde abgekühlt, das feste Salz abgesaugt und mit Diäthyläther gewaschen, i. Vak. bei 150 °C getrocknet.

Ausbeute : 48.5 g weiße Kristalle (65 % d. Th.) XVIIIa

Fp. 282 °C

Beispiel 10b

Nach der im Beispiel 10a angegebenen Vorschrift wurde das in Tab. 3 aufgeführte Phosphoniumsalz der Formel XVIIIb hergestellt.

Tabelle 3

$R^3$ = H

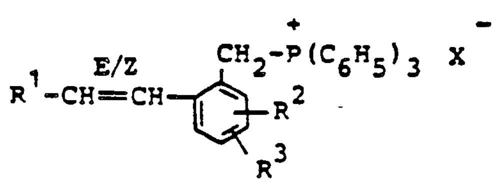

(XVIII)

| $R^1$ | $R^2$ | X | Beispiel 10 | Schmp. °C | Ausbeute % |
|---|---|---|---|---|---|
| 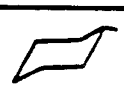 | o-Cl | Cl | b | 215 | 66 |

Beispiel 11

Arbeitsvorschrift zur Darstellung der Phosphoniumsalze der allg. Formel II (Z = $CH_2$—$CH_2$—)

Beispiel 11a

($R^1$ = Cyclohexyl, X = Cl, Z = $CH_2$—$CH_2$) 2-(2-(Cyclohexyl)-ethyl)-benzyl-triphenylphosphoniumchlorid IIa

24.85 (0.05 Mol) 2(2-Cyclohexyl)-ethenyl-benzyl-triphenylphosphoniumchlorid (Beispiel 10a) werden in 300 ml abs. Methanol gelöst, unter Stickstoff werden 5 g Palladiumtierkohle 10 % zugegeben, und in einer Schüttelente ca. 3 Stunden bis zur theoretischen $H_2$-Aufnahme (0.05 Mol $H_2$) hydriert. Über ein Klärschichtfilter mit Kieselgel wird abfiltriert, mit Methanol nachgewaschen. Das Filtrat wird i. Vak. eingeengt. Das zurückbleibende Öl wird in 50 ml Isopropanol gelöst und das Phosphoniumsalz durch Zugabe von Diäthyläther ausgefüllt und abgesaugt, anschließend wird im Vak. bei 150 °C getrocknet.

Ausbeute : 20.9 g weiße Kristalle (84 % d. Th.) IIa
($Z = CH_2—CH_2$)
Fp 241 °C

Beispiel 11b

Nach der in Beispiel 11a angegebenen Vorschrift wurde das in Tab. 4 aufgeführte Phosphoniumsalz der Formel IIb (Z = $CH_2$—$CH_2$—) hergestellt.

Tabelle 4

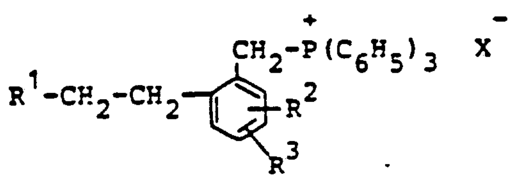

II (Z = —$CH_2$—$CH_2$—)

$R^3$ = H

| $R^1$ | $R^2$ | X | Beispiel 11 | Schmp. °C | Ausbeute % |
|---|---|---|---|---|---|
| 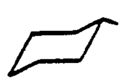 | o-Cl | Cl | b | 225 | 80 |

Herstellung des chiralen Aldehyds III

Beispiel 12

6S-Formyl-4R(tert.-Butyldiphenyl)-silyloxy-2-methoxy-3,4,5,6-tetrahydro-2,4-pyran, III

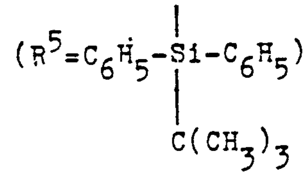

10 g (24.96 mMol) opt. aktiver « Compactinalkohol » ((+)6S-Hydroxy-methyl-4R(tert.-Butyldiphenyl)-silyloxy-2-methoxy-3,4,5,6-tetrahydro-2H-pyran (hergestellt nach Yuh-Ling Yang und J. R. Falck, Tetrahedron Letters Vol. 23, 4305 (1982)) wurden unter Rühren bei Raumtemperatur zu einer bei 0 °C bereiteten Mischung von 25 g (0.125 Mol) Chromtrioxid in 965 ml abs. $CH_2CH_2$ und 39.5 g (0.5 Mol) Pyridin abs. innerhalb 30′ zugetropft und 30′ weitergerührt. Dann wurde der Kolbeninhalt über eine Klärschicht aus Kieselgel rasch abgesaugt. Das klare Filtrat wurde in Vakuum eingeengt.

Ausbeute : 9.46 g (95 % d. Th.) III
$R_f$-wert : 0.10
Laufmittel : Cyclohexan : Essigester = 4 : 1
$C_{23}H_{30}SiO_4$ (398.6)
$^1$H-60 MHz-NMR : —CH=O  δ = 9.65 ppm
in $CDCl_3$ —$OCH_3$  δ = 3.5 ppm

Herstellung der Endprodukte I bzw. Ia

## Beispiel 13

E/Z-Epimere
6S-(2-(2-(2-(Cyclohexyl)ethyl)phenyl)-ethenyl)-4R-(tert.-Butyldiphenyl)silyloxy-2-methoxy-3,4,5,6-tetrahydro-2H-pyran, IVa Tab. 5

($R^1$=Cyclohexyl, $R^2$, $R^3$=H, $R^5$=$C_6H_5$-Si-$C_6H_5$, Z=$(CH_2)_2$
C$(CH_3)_3$

7.7 g (15.5 mMol)
2(2-(Cyclohexyl)-ethyl)-benzyl-triphenylphosphoniumchlorid (Beispiel 11a) wurden bei 100 °C i. Hochvak. getrocknet und unter Feuchtigkeitsausschluß in 67 ml abs. THF aufgeschlämmt. Bei 0 °C wurden 9.7 ml (15.5 mMol) Butyllithium in Hexan zugetropft. Man rührte ca. 30 Minuten bei 0 °C. Dann wurden ebenfalls bei 0 °C 6.12 g (15.35 mMol) 6S-Formyl-4R(tert.-Butyldiphenyl)-silyloxy-2-methoxy-3,4,5,6-tetrahydro-2H-pyran (Beispiel 12) in 15 ml abs. Tetrahydrofuran zugetropft. Nach 1 Stunde Rühren bei Raumtemperatur wurde THF im Vak. abgezogen, der Rückstand in Essigester aufgenommen und mehrmals mit Wasser extrahiert und über $MgSO_4$ getrocknet, filtriert, eingeengt. Der Rückstand wurde auf einer Kieselgelsäule (z. B. Merck Lobar® Gr. C) mit Cyclohexan-Essigester 40 : 1 chromatographisch in die reinen E- bzw. Z-Epimere aufgetrennt.
Ausbeute : 1. Fraktion 31-184 = 5.9 g E-Epimeres
2. Fraktion 185-212 = 2.1 g Z-Epimeres 8.0 g helles Öl (90 % d. Th.) IVa
E-Epimeres von IVa : $R_f$ = 0.74 Cyclohexan/Essigester = 4 : 1
Z-Epimeres von IVa : $R_f$ = 0.69 Cyclohexan/Essigester
$C_{38}H_{50}O_3Si$  MG : 582.8

## Beispiel 13b

In analoger Weise, wie in Beispiel 13 für die Verbindung IVa beschrieben, wurde die Verbindung IVb (Tab. 5, Z = $CH_2$) aus dem Aldehyd III (Bsp. 12) und den Phosphoniumsalzen II (Tab. 1) hergestellt.

### Tabelle 5

$R^2$, $R^3$=H

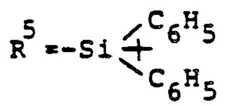

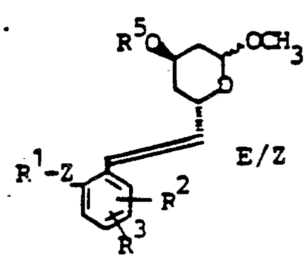

(IV)

| $R^1$ | Z | Beispiel 13 | $R_f$-wert E / Z | Ausbeute % |
|---|---|---|---|---|
| 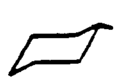 | $CH_2$ | b | 0.27/0.18[4] | 77 |

4) Toluol

Beispiel 14

E/Z-Epimere

6S-[2-(2-(2-(Cyclohexyl)ethyl)phenyl)-ethenyl]-4R-(tert.-Butyldiphenyl)silyloxy-2-hydroxy-3,4,5,6-tetrahydro-2H-pyran Va (Tab. 6)

($R^1$ = Cyclohexyl, $R^2$, $R^3$ = H, $R^5 = C_6H_5SiC_6H_5$, Z = $(CH_2)_2$)
|
$C(CH_3)_3$

3.72 g (6.4 mMol) der Verbindung IVa (Bsp. 13a) werden in 13 ml Tetrahydrofuran gelöst, dann werden 13 ml Wasser und 19 ml Eisessig zugegeben. Man rührt ca. 5 Stunden bei 70 °C, entfernt das Tetrahydrofuran i. Vak., versetzt den Rückstand mit 30 ml Toluol und engt zur azeoptropen Entfernung restlichen Eisessigs erneut i, Vak. ein.

Ausbeute : 3.2 g (89 % d. Th.) Va

$C_{37}H_{48}O_3Si$ = 568.8

$R_f$ = 0.36 E-Epimer Laufmittel : Cyclohexan/Essigester = 4 : 1

$R_f$ = 0.25 Z-Epimer

Beispiel 14b

In analoger Weise, wie in Beispiel 14 für Va beschrieben, wurde die Verbindung Vb (Tab. 6) hergestellt. Als Ausgangsverbindungen kann die entsprechenden Verbindungen IVb (Tab. 5) in Form der reinen E oder Z Epimere, oder als E/Z-Epimerengemische eingesetzt werden.

Tabelle 6

$R^2$, $R^3$=H

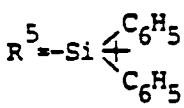

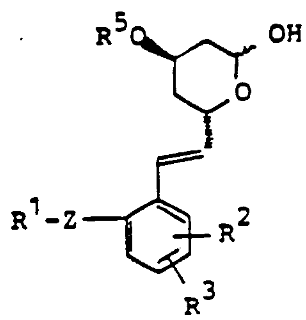

(V)

| $R^1$ | Z | Beispiel | $R_f$-wert E | A |
|---|---|---|---|---|
| 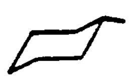 | $CH_2$ | b | 0.26 | |

Beispiel 15

E/Z-Epimere

6S-[2-(2-(2-(Cyclohexyl)-ethyl)phenyl-ethenyl]-4R-(tert.-Butyldiphenyl)silyloxy-3,4,5,6-tetrahydro-2H-pyran-2-on VI a (Tab. 7)

($R^1$ = Cyclohexyl, $R^2$, $R^3$ = H, $R^5 = C_6H_5SiC_6H_5$, Z = $(CH_2)_2$)
|
$C(CH_3)_3$

2.88 g (28.8 mMol) Chromtrioxid werden in 306 ml Methylenchlorid abs. vorgelegt und bei 0 °C bis eine feinpulvrige Aufschlämmung vorliegt gerührt. Dann werden 4.55 g (57.6 mMol) abs. Pyridin in 10 ml abs. Methylenchlorid zugetropft. Die orangegelbe Lösung wird 20 Minuten gerührt. Anschließend gibt man 1.64 (2.88 mMol) der Verbindung V a (Beispiel 14) gelöst in 10 ml abs. Methylenchlorid tropfenweise zu. Man rührt noch 30′ bei Raumtemperatur. Die Reaktionslösung wird über eine Klärschicht aus Kieselgel und $MgSO_4$ abgesaugt und i. Vak. eingeengt.

Ausbeute : 1.14 g (70 % d. Th.) (E/Z-Epimere) VIa

$C_{37}H_{46}O_3Si$ 566.8

$R_f$ = 0.34 E-Epimer Laufmittel : Cyclohexan/Essigester = 4 : 1
$R_f$ = 0.30 Z-Epimer

Zur Herstellung des reinen E (trans)-Epimeren der Verbindung VIa wurden 0.52 g des E/Z-Epimerengemischs von VIa in 10 ml Toluol gelöst und mit 52 mg Jod versetzt und anschließend 48 Stunden am Rückfluß erhitzt. Nach dem Entfernen des Toluols i. Vak. und Filtration des Rückstands über Kieselgel (Laufmittel : Cyclohexan/Essigester = 4 : 1) erhielt man das E-Epimere von VI a.

Ausbeute : 0.42 g VIa-E-Epimer (81 % d. Th.)
$R_f$ = 0.33 (Cyclohexan/Essigester 4 : 1)

## Beispiel 15b

In analoger Weise, wie in Beispiel 15 für die Verbindung VIa beschrieben, wurde die Verbindung VIb (Tab. 7) hergestellt. Als Ausgangsverbindungen kann die entsprechenden Verbindung Vb (Tab. 6) in Form der reinen E oder Z-Epimere, oder als E/Z-Epimerengemischen eingesetzt werden. Die reinen E-Epimere erhält man, wie oben beschrieben, leicht durch Isomerisierung der E/Z-Epimerengemische in Gegenwart von Jod, oder durch chromatographische Trennung.

Tabelle 7

$R^2$, $R^3$ = H

$R^5$ = 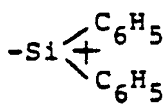

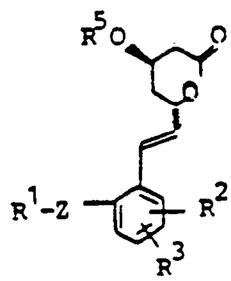

(VI)

| $R^1$ | Z | Beispiel 15 | $R_f$-wert 1) E/Z | Ausbe % |
|---|---|---|---|---|
| 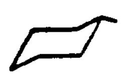 | $CH_2$ | b | 0.26[1] | 68 |

1) Cyclohexan/EtOAc 4 : 1

## Beispiel 16

Herstellung optisch reiner Verbindungen der allg. Formel I : (+)-E-6S-[2-(2-(2-(Cyclohexyl)ethyl)phenyl-ethenyl]-4R-hydroxy-3,4,5,6-tetrahydro-2H-pyran-2-on I Tab.

($R^1$ = Cyclohexyl, $R^2$, $R^3$ = H, A-B = CH = CH, Z = $CH_2$—$CH_2$)

0.47 g (0.83 mMol) der Verbindung VIa (E-Epimer) (Bsp. 15a) werden in 50 ml Tetrahydrofuran gelöst mit 0.1 ml (1.7 mMol) Eisessig und 0.39 g (1.24 mMol) Tetrabutylammoniumfluorid $\cdot 3H_2O$ versetzt. Man rührt bei 20 °C ca. 5 Stdn.. Dann wird das Lösungsmittel i. Vak. entfernt und der Rückstand mit Diäthyläther aufgenommen. Die org. Phase wird 1 × mit Wasser und 1 × mit gesättigter $NaHCO_3$-Lösung extrahiert, dann wird über $MgSO_4$ getrocknet, filtriert und i. Vak. eingeengt. Über eine Kieselgelsäule (z. B. Merck Lobar ®) und Cyclohexan/Essigester = 1 : 1 chromatographiert ergab :

Ausbeute : 0.19 g (72 % d. Th.) Ia
$C_{21}H_{28}O_3$ MG : 328
$R_f$-wert = 0.18 (Cyclohexan/Essigester 1 : 1)
$^1H$ 270 MHz NMR : δ-werte in ppm : (vgl : Tab. 8)

## Beispiele : 16a-16i

In analoger Weise, wie in Beispiel 16 für die Verbindung Ia beschrieben, wurden die Verbindungen Ib-Ii (Tab. 8) hergestellt.

Sofern Vorprodukte der in Tab. 8 aufgeführter Endprodukte nicht beschrieben wurden, wurden sie in anologer Weise wie in den vorhergehenden Beispielen erläutert, erhalten.

Tabelle 8

$R^2$, $R^3$=H

(A-B=-CH=CH-)

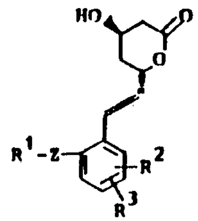

(I)

| $R^1$ | Z | Beispiel 16 | $R_f$-wert 1) E | Ausbeute % | $^1$H-270 MH$_2$-NMR δ-Werte in ppm. | Massenspektrum |
|---|---|---|---|---|---|---|
| 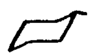 | $CH_2$-$CH_2$ | a | 0.18 | 72 | 4.4-4.48 (m, 1H) CHOH, 5.3-5.4 (m, 1H) CHOCO, 6,12 (dd, J=16 Hz, 1H) CH=6.93 (d, J=16Hz, 1H) CH= 7.05-7,55 (m, 4H) aromat. Prot. | $C_{21}H_{28}O_3$ M=328 |

| $R^1$ | Z | Beispiel 16 | $R_f$-wert 1) Fp. °C E | Ausbeute % | $^1$H-279 NH$_2$-NMR δ-Werte in ppm. | Massenspektrum |
|---|---|---|---|---|---|---|
| 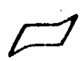 | $CH_2$ | b | 0.18[1] | 94 | 4.4-4.5 (m, 1H) CHOH; 5.3-5.4 (m, 1H) CHOCO 6.1 (dd, J=16Hz, 1H) CH=; 6.95 (d, J=16Hz, 1H) CH=; 7.1-7.5 (m, 4H) aromat. Prot. | $C_{20}H_{26}O_3$ M=314 |

Tabelle 8 Fortsetzung

(A-B = CH = CH-)

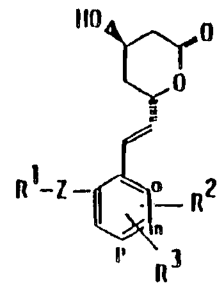

| $R^1$ | $R^2$ | $R^3$ | Z | Beispiel 16 | $R_f$-Wert/Drehwert 1) E | Ausbeute % | $^1H$-270 MHz-NMR δ-Werte in ppm. | Massenspektrum/Fp. |
|---|---|---|---|---|---|---|---|---|
| 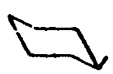 | o-$CH_3$ | H | $CH_2CH_2$ | c | 0,43<br>[α] = +29,4°<br>$CH_3OH$<br>(C = 1) | 43 | 0,85 - 2,2 (m, 16H)Cyclohexyl, $CH_2$, OH); 2,29 (s, 3H) $CH_3$; 2,55-2,9 (m,4H) $CH_2$,$CH_2$; 4,45-4,52 (m, 1H)CHOCO; 5,7(dd, J = 16Hz, 1H)CH= ; 6,72 (d, J = 16Hz, 1H)CH=;7,0-7,2 (m,3H) aromat. Prot. | $C_{22}H_{30}O_3$<br>343,12<br>Fp.: 89°C |
| 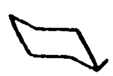 | o-Cl | H | $CH_2CH_2$ | d | 0,45 | 38 | 0,85-2,2 (m, 16H)Cyclohexyl, $CH_2$, OH; 2,6-2,95 (m, 4H)$CH_2$,$CH_2$; 4,45 -- 4,55 (m, 1H) CHOH; 5,35-5.45 (m, 1H)CHOCO; 5,9 (dd, J = 16Hz, 1H)CH=; 7,05 - 7,25 (m, 3H) aromat Prot. | $C_{21}H_{27}O_3Cl$<br>363,57<br>Fp.: 68°-69°C |

Tabelle 8 Fortsetzung

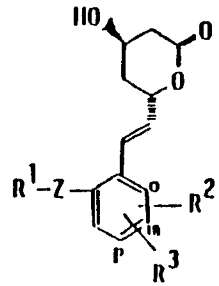

(A-B = CH = CH-)

| $R^1$ | $R^2$ | $R^3$ | Z | Beispiel 16 | 1) $R_f$-Wert/Drehwert E | Ausbeute % | $^1H$-270 MHz-NMR δ-Werte in ppm. | Massenspektrum/Fp. |
|---|---|---|---|---|---|---|---|---|
| 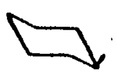 | o-Cl | p-Cl | $CH_2CH_2$ | e | 0,4 | 45 | 0,85-2,2 (m, 16H)Cyclohexyl, $CH_2$, OH; 2,6-2,94 (m, 4H) $CH_2$, $CH_2$; 4,4-4,56 (m, 1H)CHOH; 5,35-5,45 (m,1H)CHOCO; 5,87 (dd, J = 16Hz, 1H)CH=; 6,5 (d, J = 16Hz, 1H)CH=;7,06 (d, 1H); 7,26 (d, 1H) aromat. Prot. | $C_{21}H_{26}O_3Cl_2$ 398,02 |

Tabelle 8 Fortsetzung

(A-B = CH = CH-)

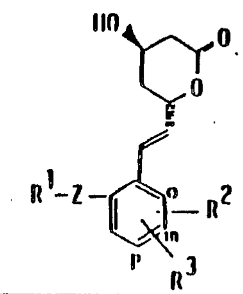

| $R^1$ | $R^2$ | $R^3$ | Z | Beispiel 16 | 1) $R_f$-Wert/Drehwert E | Ausbeute % | $^1H$-270 MHz-NMR δ-Werte in ppm. | Massenspektrum/Fp. |
|---|---|---|---|---|---|---|---|---|
| 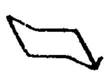 | o-Cl | p-Cl | $CH_2$ | f | 0,22<br>[α] =+19.2°<br>c=1, MeOH | 86 | 0,85-1,8 (m, 12H)Cyclohexyl, OH; 1,9-2,2 (m,2H) $CH_2$; 2,5 (d,J = 7Hz, 2H) $CH_2$; 2,65-2,9 (m, 2H)$CH_2$; 4,4-4,5(m, 1H)CHOH; 5,35-5,45 (m, 1H)CHOCO; 5,88 (dd, J = 16Hz, 1H)CH=; 6,58 (d,J = 16Hz, 1H)CH=; 7,06(d,1H), 7,26 (d,1H) aromat. Prot. | $C_{20}H_{24}Cl_2O_3$<br>383,3<br>Fp.: 100-102°C |

Tabelle 8 Fortsetzung

(A-B = CH = CH-)

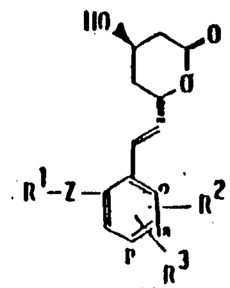

| $R^1$ | $R^2$ | $R^3$ | Z | Beispiel 16 | $R_f$-Wert/Drehwert[1)] E | Ausbeute % | $^1$H-270 MHz-NMR δ-Werte in ppm. | Massenspektrum/Fp. |
|---|---|---|---|---|---|---|---|---|
| 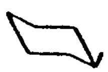 | o-$CH_3$ | H | $CH_2$ | g | 0,2 | 93 | 0,85-1,0 (m, 1H) Cyclohexyl OH; 1,95-2,2 (m, 2H) $CH_2$; 2,29 (s, 1H) $CH_3$; 2,49 (d, J = 7Hz, 1H) $CH_2$; 2,65-2,9 (m, 2H) $CH_2$; 4,45-4,53 (m 1H) CHOCO; 5,7 (dd, J = 16Hz, 1H) CH= ; 6,7 (d, J = 16Hz, 1H) CH=; 6,95 - 7,15 (m, 3H) aromat. Prot. | $C_{21}H_{28}O_3$ 328,43<br>Fp. :99 -101°C |

Tabelle 8 Fortsetzung

(A-B = CH = CH-)

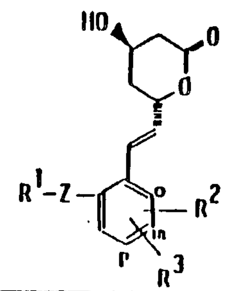

| $R^1$ | $R^2$ | $R^3$ | Z | Beispiel 16 | $R_f$-Wert/Drehwert 1) E | Ausbeute % | $^1$H-270 MHz-NMR δ-Werte in ppm. | Massenspektrum/Fp. |
|---|---|---|---|---|---|---|---|---|
| 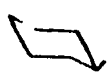 | o-Cl | H | $CH_2$ | h | 0,2 | 98 | 0,85-1,8 (m, 12H) Cyclohexyl, OH; 1,95-2,25 (m, 2H) $CH_2$; 2,55 (d, J = 7Hz, 1H) $CH_2$; 2,6-2,9 (m, 2H) $CH_2$; 4,45-4,53 (m, 1H) CHOH; 5,35-5,46 (m, 1H) CHOCO; 5,9 (dd, J = 16Hz, 1H) CH=; 6,65 (d, J = 16Hz, 1H) CH=; 7,05-7,3 (m, 3H) aromat. Prot.; | $C_{20}H_{25}ClO_3$ 348,86 |

Tabelle 8 Fortsetzung

(A-B = CH = CH-)

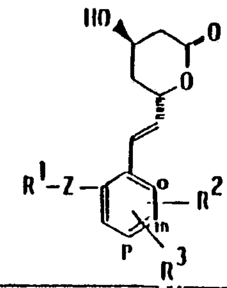

| $R^1$ | $R^2$ | $R^3$ | Z | Beispiel 16 | $R_f$-Wert/Drehwert[1] E | Ausbeute % | $^1$H-270 MHz-NMR δ-Werte in ppm. | Massenspektrum/Fp. |
|---|---|---|---|---|---|---|---|---|
| 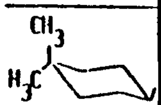 | o-Cl | p-Cl | $CH_2$ | i | 0,24 | 69 | 0,85-1,8 (m, 10H)$CH_2$,OH; 1,9-2,2 (m, 2H)$CH_2$; 0,89 (2 s, 6H)$CH_3$; 2,52 (d, J = 7Hz, 2H)$CH_2$; 2,64-2,86 (m, 2H)$CH_2$; 4,41-4,52 (m 1H)CHOH; 5,33-5,44 (m, 1H)CHOCO; 5,87 (dd, J = 16Hz, 1H)CH=; 6,57 (d, J = 16Hz, 1H)CH=; 7,06 (d, 1H); 7,25 (d, 1H) aromat. Prot. | $C_{22}H_{28}O_3Cl_2$ 411,35 |

[1] Cyclohexan/Essigsäureethylester = 1 : 1

Beispiel 17

(+)-6S-[2-(2-(2-(Cyclohexyl)ethyl)phenyl-ethyl]-4R-hydroxy-3,4,5,6-tetrahydro-2H-pyran-2-on I'a Tab. 9
($R^1$ = Cyclohexyl, $R^2$, $R^3$ = H, A-B = $CH_2—CH_2$, Z = $CH_2—CH_2$)

1g Pd/C 10 % werden in 20 ml abs. $CH_3OH$ in einer Schüttelente vorhydriert. Dann werden 0.12 g (0.38 mMol) der Verbindung Ia (Beispiel 16) gelöst in 10 ml abs. MeOH zugegeben. Man hydriert bei Raumtemperatur. Nach Aufnahme von 10 ml $H_2$ (109.) wird die Hydrierung abgebrochen, der Katalysator abgesaugt und das Filtrat in Vakuum eingeengt.

Ausbeute : 0.11 g (91 % d. Th.) I' a (A-B = $CH_2—CH_2$)

$R_f$ = 0.30 (Laufmittel : Cyclohexan/Essigester 1 : 1)

$^1H$ 270 MHz NMR δ-werte in ppm : (vgl. Tab. 9)

Beispiele 17a-17b

In analoger Weise, wie in Beispiel 17 für die Verbindung Ia' beschrieben, können die Verbindungen I (A-B = —CH = CH—) hydriert werden, zu den Verbindungen I' (A-B = $CH_2—CH_2$) (Tab. 9)

(Siehe Tabelle 9 Seite 29 ff.)

Beispiel 18

Herstellung der Salze der freien Dihydroxysäuren der Verbindungen der allg. Formel Ia
(+)-(E)-(3R,5S)-7[2-(2-(Cyclohexyl)ethyl)phenyl]-3,5-dihydroxy-6-heptensäure-Kaliumsalz
($R^1$ = —⊙—F, Z = $CH_2—CH_2$, $R^2$, $R^3$ = H, $R^4$ = $K^{\oplus}$), A-B = —CH = CH—

0.1 g der Verbindung I a (Bsp. 16) werden in 5 ml abs. EtOH gelöst. Zu dieser Lösung werden bei Raumtemp. 2,9 ml einer 0.1 molaren Lösung von KOH in EtOH zugegeben. Nach ca. 3 Stunden ist im Dünnschichtchromatogramm (Laufmittel Cyclohexan/Essigester 1 : 1) kein Ester mehr vorhanden. Die äthanolische Lösung wird in Vakuum eingeengt. Zurück bleiben

Ausbeute : 119 mg weiße Kristalle des K-salzes

IR : C = O-Bande 1608/1572 $cm^{-1}$

In analoger Weise können, wie in Beispiel 18 beschrieben, die Kaliumsalze der entsprechenden freien Dihydroxysäuren der Verbindungen der allg. Formel Ia erhalten werden.

Beispiel 19

Herstellung des Methylesters der freien Dihydroxysäuren der allg. Formel Ia
(+)-(E)-(3R,5S)-7[2-(2-(Cyclohexyl)ethyl)phenyl-3,5-dihydroxy-6-heptensäure-methylester
($R^1$ = c-$C_6H_{11}$, Z=—$CH_2—CH_2$, $R^2$, $R^3$ = H, $R^4$ = $CH_3$), A-B = —CH = CH—

0.4 g der Verbindung Ia (Beispiel 16) wurden in 10 ml abs. MeOH gelöst. Zu dieser Lösung wurden bei Raumtemperatur 1.3 ml einer 0.1 molaren Lösung von $NaOCH_3$ in abs. MeOH zugegeben und ca. 1 Stunde gerührt. Dann wurde das Lösungsmittel i. Vak. entfernt und der Rückstand in Wasser aufgenommen, es wurde bei 0 °C auf pH = 7 gestellt und mit Essigester rasch extrahiert, mit $MgSO_4$ getrocknet, filtriert und das Lösungsmittel i. Vak. entfernt. Zurück blieb die Titelverbindung.

Ausbeute : 0.35 g Methylester

$R_f$ = 0.21 (Laufmittel : Cyclohexan/Essigester = 1 : 1)

$^1H$-60 MHz-NMR δ-wert in ppm.

3.69 (S,3H)-$OCH_3$

In analoger Weise können, wie in Beispiel 19 beschrieben, die Methylester der freien Dihydroxysäuren der allg. Formel Ia hergestellt werden. Durch Ersatz von Methanol durch andere Alkohole ($R^4$-OH) sind auch entsprechende andere Ester Ia($R^4$ = $C_2H_5$, i-$C_3H_7$, Benzyl etc.) leicht darstellbar.

(Siehe Patentansprüche Seite 31 f.)

Tabelle 9

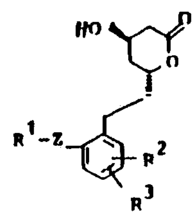

(I')

$R^2$, $R^3$ = H

(A-B = -$CH_2$-$CH_2$-)

| $R^1$ | Z | Beispiel 17 | $R_f$-Wert [1] F. | Ausbeute % | $^1H$ 270 MHz NMR ($CDCl_3$) δ-Werte in ppm | Massenspektrum |
|---|---|---|---|---|---|---|
| 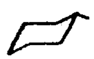 | -$CH_2$-$CH_2$- | a | 0.30 | 91 | 1.2-3.0 (m, 24H) CH, $CH_2$, OH; 4.38-4.46 (m, 1H) -CH-OH; 4.7-4.8 (m, 1H) -CH-OCO; 7.1 (s, 4H) aromat. Protonen | $C_{21}H_{30}O_3$ M=330 M-$H_2O$=312 |

Tabelle 9 Fortsetzung

$R^2$, $R^3$=H

(A-B=-$CH_2$-$CH_2$-)

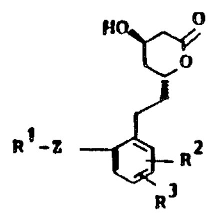

I'

| $R^1$ | Z | Beispiel 17 | $R_f$-Wert E | Ausbeute % | $^1$H-270 MHz NMR ($CDCl_3$) δ-Werte in ppm | Massenspektren |
|---|---|---|---|---|---|---|
| 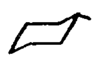 | $CH_2$ | b | 0.19 [3] | 97 | 0.9-1.3(m,5H)$CH_2$,CH;1.4-2.0(m,11H)$CH_2$, CH;2.5(d,J=8Hz,2H)$CH_2$;2.6-3.0(m,4H)$CH_2$; 4.35-4.45(m,1H) CHOH;4.65-4.8(m,1H) CHOCO;7.1-7.2(m,4H)aromat. Protonen | $C_{20}H_{28}O_3$ M=316 |

**Patentansprüche** (für die Vertragsstaaten : BE, CH, DE, FR, GB, IT, LI, LU, NL, SE)

1. 3-Desmethyl-mavalonsäurederivate der Formel I (δ-Lacton) bzw. Ia (entsprechendes Dihydroxycarbonsäurederivat)

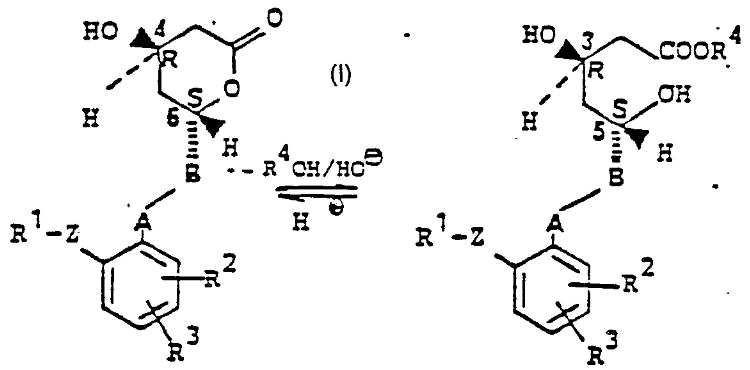


(I) (Ia)

worin bedeuten :

A-B einen Rest der Formel —CH=CH— oder $—CH_2—CH_2—$,

Z eine $—CH_2—$ oder $—CH_2—CH_2$-Gruppe,

$R^1$ einen cycloaliphatischen Kohlenwasserstoff mit 3 bis 7 Kohlenstoffatomen, der gegebenenfalls mit 1 oder 2 Methylgruppen substituiert ist,

$R^2$, $R^3$ Wasserstoff, Halogen, Trifluoromethyl, Alkyl oder Alkoxy mit je 1-6 C-Atomen,

$R^4$ Wasserstoff oder einen geradkettigen oder verzweigten $C_1$-$C_5$-Alkylrest, Benzyl, 1-2 fach mit $C_1$-$C_4$-Alkyl oder Halogen substituiertes Benzyl, Alkalimetall oder Ammonium ($NH_4^+$) oder mit $C_1$-$C_4$-Alkyl oder Hydroxy-$C_1$-$C_4$-alkyl substituiertes Ammoniumion.

2. Verbindungen gemäß Anspruch 1, dadurch gekennzeichnet, daß in Formel I bzw. Ia

$R^1$ Cyclopentyl oder Cyclohexyl ist,

$R^2$, $R^3$ Wasserstoff, Halogen, Trifluormethyl, Alkyl oder Alkoxy mit je 1 bis 4 C-Atomen bedeuten und

$R^4$ Wasserstoff, Methyl, Äthyl, Isopropyl, Isobutyl, Benzyl, Natrium, Kalium, Ammonium, Trishydroxymethyl-methylamin darstellt.

3. Verbindungen gemäß Anspruch 1, dadurch gekennzeichnet, daß in Formel I bzw. Formel Ia

$R^1$ Cyclopentyl, Cyclohexyl

$R^2$, $R^3$ Wasserstoff, 2-Methyl, 2-Trifluormethyl, 2,4-Dimethyl, 2-Methyl-4-chlor, 2-Chlor-4-methyl, 2,4-Bistrifluormethyl, 2-Äthyl, 2-Isopropyl, 2-Isobutyl, 2-Chlor, 2-Fluor, 2-Brom, 2,4-Dichlor, 2,4-Difluor, 2-Methoxy, 4-Methoxy, 2,4-Dimethoxy bedeuten und

$R^4$ Wasserstoff, Methyl, Äthyl, Benzyl, Natrium, Kalium, Ammonium, Trishydroxymethyl-methylamin darstellt.

4. Verfahren zur Herstellung von Verbindungen der Formeln I und Ia, dadurch gekennzeichnet, daß man

a) die Phosphoniumsalze der Formel II

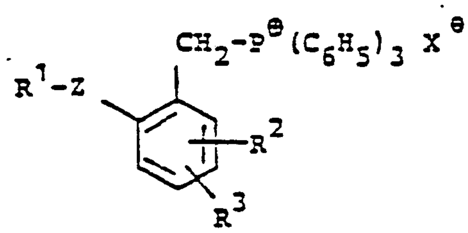

(II)

worin $R^1$, $R^2$, $R^3$ und Z die zur Formel I angegebene Bedeutung haben und X = Cl, Br, J ist, mit dem chiralen Aldehyd der Formel III umsetzt,

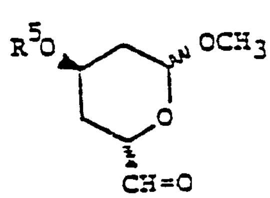

(III)

worin $R^5$ eine gegen Basen und schwache Säuren stabile Schutzgruppe bedeutet, zu einer Verbindung der Formel

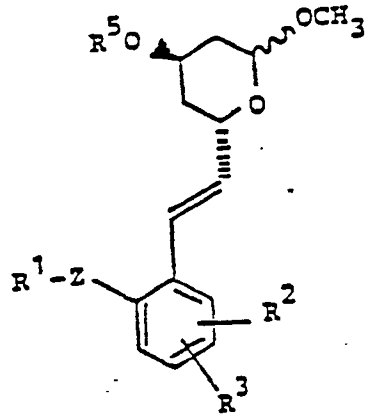

(IV)

worin $R^1$, $R^2$, $R^3$ und Z die zur Formel I, $R^5$ die zur Formel III gegebene Bedeutung haben (und AB die (—CH=CH—)-Gruppe darstellt),

b) in einer Verbindung der allg. Formel IV die Methylacetalfunktion sauer hydrolysiert zu einem Lactol der Formel V,

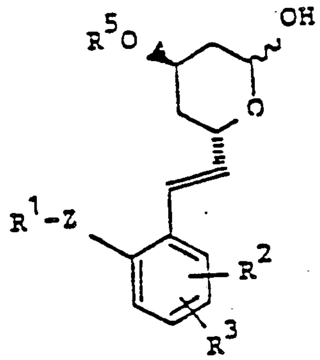

(V)

worin $R^1$, $R^2$, $R^3$ und Z die Formel I, $R^5$ die zur Formel III gegebene Bedeutung hat (und A-B die (—CH=CH—)-Gruppe darstellt),

c) die Verbindung der allg. Formel V oxydiert zu einem Lacton der Formel VI,

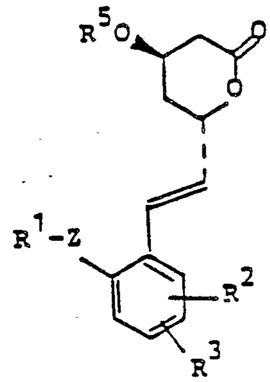

(VI)

worin $R^1$, $R^2$ und $R^3$ und Z die zur Formel I, $R^5$ die zur Formel III gegebene Bedeutung hat (und A-B die (—CH=CH—)-Gruppe darstellt),

d) in einer Verbindung der allg. Formel VI die Schutzgruppe $R^5$ abspaltet zu einer Verbindung der Formel I, worin $R^1$, $R^2$, $R^3$ und Z die zur Formel I angegebenen Bedeutungen haben und A-B die (—CH=CH—)-Gruppe darstellt, gegebenenfalls eine Verbindung der allgemeinen Formel I bei der A-B eine (—CH=CH—) Gruppe darstellt, hydriert zu einer Verbindung der allgemeinen Formel I, in der A-B eine (—$CH_2$—$CH_2$—)-Gruppe darstellt, wobei die Hydrierung auch bei den Verbindungen der Formeln IV, V oder VI erfolgen kann zu entsprechenden Verbindungen, worin A-B die (—$CH_2$—$CH_2$—)-Gruppe darstellt, gegebenenfalls ein Hydroxylacton I in die entsprechenden freien Hydroxysäuren Ia bzw. deren Salze überführt oder gegebenenfalls den entsprechenden Ester aus den freien Hydroxysäuren Ia bzw. aus dem Hydroxylacton I darstellt.

5. Pharmazeutisches Präparat, dadurch gekennzeichnet, daß es eine Verbindung gemäß Anspruch 1 enthält.

6. Verwendung von Verbindungen gemäß Anspruch 1 zur Prophylaxe und Therapie der Arteriosklerose und Hypercholesterinämie.

7. Verbindungen der Formel IV

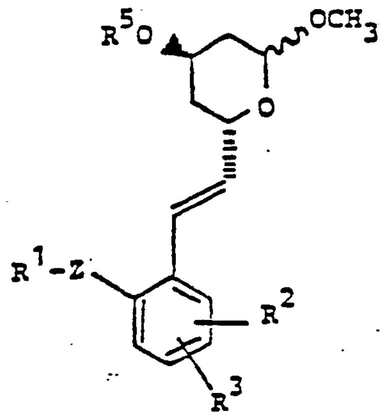

(IV)

worin $R^1$, $R^2$, $R^3$ und Z die zu Formel I und $R^5$ die zu Formel III angegebenen Bedeutungen haben.

8. Verbindungen der Formel V

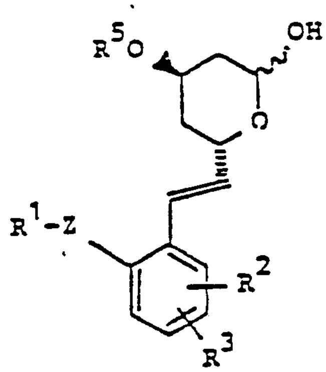

(V)

worin $R^1$, $R^2$, $R^3$ und Z die zu Formel I und $R^5$ die zu Formel III angegebenen Bedeutungen haben.

9. Verbindungen der Formel VI

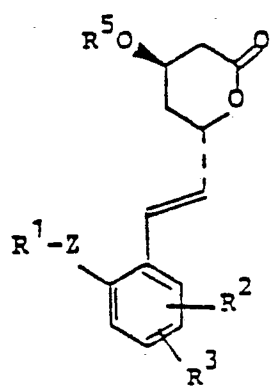

(VI)

worin $R^1$, $R^2$, $R^3$ und Z die zu Formel I und $R^5$ die zu Formel III angegebenen Bedeutungen haben.

**Patentansprüche** (für den Vertragsstaat AT)

1. Verfahren zur Herstellung von 3-Desmethyl-mevalonsäurederivaten der Formel I (δ-Lacton) bzw. Ia (entsprechendes Dihydroxycarbonsäurederivat)

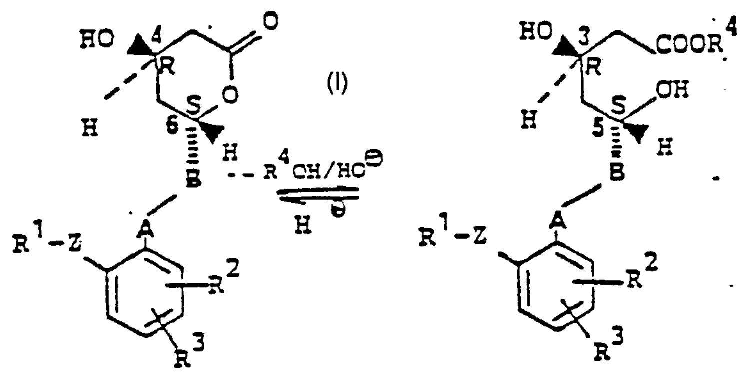


(I) (Ia)

worin bedeuten :

A-B einen Rest der Formel —CH=CH— oder —$CH_2$—$CH_2$—

Z eine —$CH_2$— oder —$CH_2$—$CH_2$-Gruppe,

$R^1$ einen cycloaliphatischen Kohlenwasserstoffrest mit 3 bis 7 Kohlenstoffatomen, der gegebenenfalls mit 1 oder 2 Methylgruppen substituiert ist,

$R^2$, $R^3$ Wasserstoff, Halogen, Trifluoromethyl, Alkyl oder Alkoxy mit je 1-6 C-Atomen,

$R^4$ Wasserstoff oder einen geradkettigen oder verzweigten $C_1$-$C_5$-Alkylrest, Benzyl, 1-2 fach mit $C_1$-$C_4$-Alkyl oder Halogen substituiertes Benzyl, Alkalimetall oder Ammonium ($NH_4^+$) oder mit $C_1$-$C_4$-Alkyl oder Hydroxy-$C_1$-$C_4$-alkyl substituiertes Ammoniumion, dadurch gekennzeichnet, daß man

a) die Phosphoniumsalze der Formel II

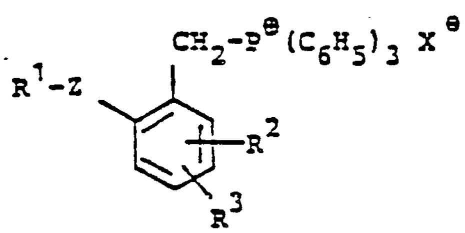

(II)

worin $R^1$, $R^2$, $R^3$ und Z die zur Formel I angegebene Bedeutung haben und X = Cl, Br, J ist, mit dem chiralen Aldehyd der Formel III umsetzt,

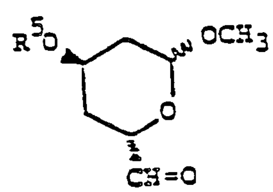

(III)

worin $R^5$ eine gegen Basen und schwache Säuren stabile Schutzgruppe bedeutet, zu einer Verbindung der Formel

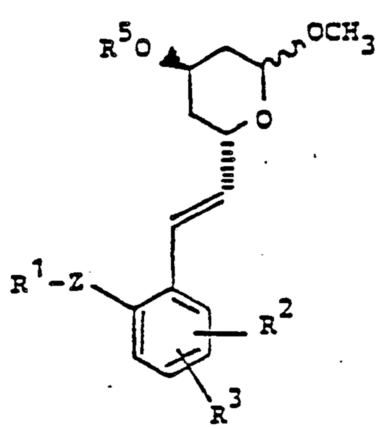

(IV)

worin $R^1$, $R^2$, $R^3$ und Z die zur Formel I, $R^5$ die zur Formel III gegebene Bedeutung haben (und AB die (—CH = CH—)-Gruppe darstellt),

b) in einer Verbindung der allg. Formel IV die Methylacetalfunktion sauer hydrolysiert zu einem Lactol der Formel V,

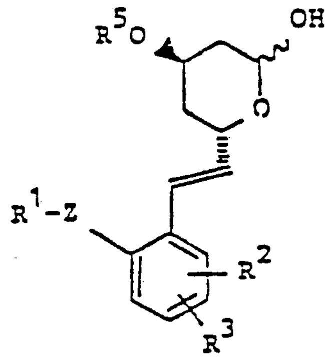

(V)

worin $R^1$, $R^2$, $R^3$ und Z die Formel I, $R^5$ die zur Formel III gegebene Bedeutung hat (und A-B die (—CH = CH—)-Gruppe darstellt),

c) die Verbindung der allg. Formel V oxydiert zu einem Lacton der Formel VI,

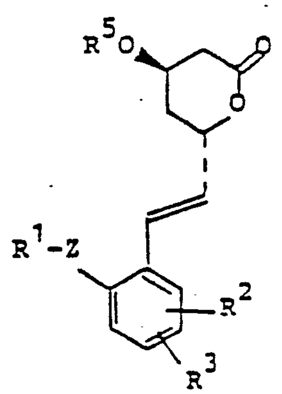

(VI)

worin $R^1$, $R^2$ und $R^3$ und Z die zur Formel I, $R^5$ die zur Formel III gegebene Bedeutung hat (und A-B die (—CH = CH—)-Gruppe darstellt),

d) in einer Verbindung der allg. Formel VI die Schutzgruppe $R^5$ abspaltet zu einer Verbindung der Formel I, worin $R^1$, $R^2$, $R^3$ und Z die zur Formel I angegebenen Bedeutungen haben und A-B die (—CH=CH—)-Gruppe darstellt, gegebenenfalls eine Verbindung der allgemeinen Formel I bei der A-B eine (—CH=CH—) Gruppe darstellt, hydriert zu einer Verbindung der allgemeinen Formel I, in der A-B eine ($—CH_2—CH_2—$)-Gruppe darstellt, wobei die Hydrierung auch bei den Verbindungen der Formeln IV, V oder VI erfolgen kann zu entsprechenden Verbindungen, worin A-B die ($—CH_2—CH_2—$)-Gruppe darstellt, gegebenenfalls ein Hydroxylacton I in die entsprechenden freien Hydroxysäuren Ia bzw. deren Salze überführt oder gegebenenfalls den entsprechenden Ester aus den freien Hydroxysäuren Ia bzw. aus dem Hydroxylacton I darstellt.

2. Verfahren gemäß Anspruch 1, dadurch gekennzeichnet, daß man eine Verbindung der Formel I bzw. Ia, worin

$R^1$ Cyclopentyl oder Cyclohexyl ist,

$R^2$, $R^3$ Wasserstoff, Halogen, Trifluormethyl, Alkyl oder Alkoxy mit je 1 bis 4 C-Atomen bedeuten und

$R^4$ Wasserstoff, Methyl, Äthyl, Isopropyl, Isobutyl, Benzyl, Natrium, Kalium, Ammonium, Trishydroxymethyl-methylamin darstellt, herstellt.

3. Verfahren gemäß Anspruch 1, dadurch gekennzeichnet, daß man eine Verbindung der Formel I bzw. Formel Ia, worin

$R^1$ Cyclopentyl, Cyclohexyl

$R^2$, $R^3$ Wasserstoff, 2-Methyl, 2-Trifluormethyl,2,4-Dimethyl, 2-Methyl-4-chlor, 2-Chlor-4-methyl, 2,4-Bistrifluormethyl, 2-Äthyl, 2-Isopropyl, 2-Isobutyl, 2-Chlor, 2-Fluor, 2-Brom, 2,4-Dichlor, 2,4-Difluor, 2-Methoxy, 4-Methoxy, 2,4-Dimethoxy bedeuten und

$R^4$ Wasserstoff, Methyl, Äthyl, Benzyl, Natrium, Kalium, Ammonium, Trishydroxymethyl-methylamin darstellt, herstellt.

4. 3-Desmethyl-mevalonsäurederivate der Formel I bzw. Ia gemäß Anspruch 1 erhältlich nach Verfahren gemäß Anspruch 1.

5. Verfahren zur Herstellung eines pharmazeutischen Präparates, dadurch gekennzeichnet, daß man

eine Verbindung erhältlich gemäß Anspruch 1 in eine geeignete Darreichungsform überführt.

6. Verfahren gemäß Anspruch 1, dadurch gekennzeichnet, daß man die Zwischenstufe der Formel IV

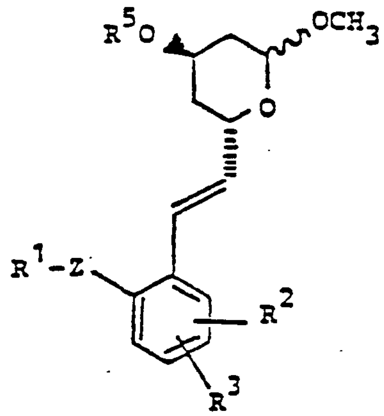

(IV)

worin $R^1$, $R^2$, $R^3$ und Z die zu Formel I und $R^5$ die zu Formel III angegebenen Bedeutungen haben, isoliert.

7. Verfahren gemäß Anspruch 1, dadurch gekennzeichnet, daß man die Zwischenstufe der Formel V

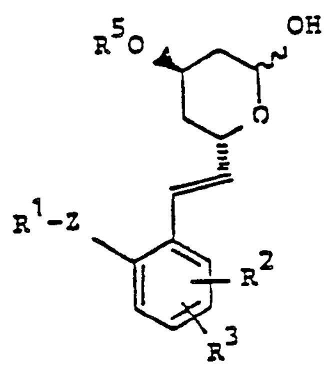

(V)

worin $R^1$, $R^2$, $R^3$ und Z die zu Formel I und $R^5$ die zu Formel III angegebenen Bedeutungen haben, isoliert.

8. Verfahren gemäß Anspruch 1, dadurch gekennzeichnet, daß man die Zwischenstufe der Formel VI

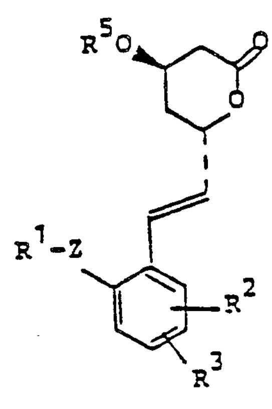

(VI)

worin $R^1$, $R^2$, $R^3$ und Z die zu Formel I und $R^5$ die zu Formel III angegebenen Bedeutungen haben, isoliert.

**Claims** (for the Contracting States : BE, CH, DE, FR, GB, IT, LI, LU, NL, SE)

1. A 3-demethylmevalonic acid derivative of the formula I (δ-lactone) or Ia (corresponding dihydroxy carboxylic acid derivative)

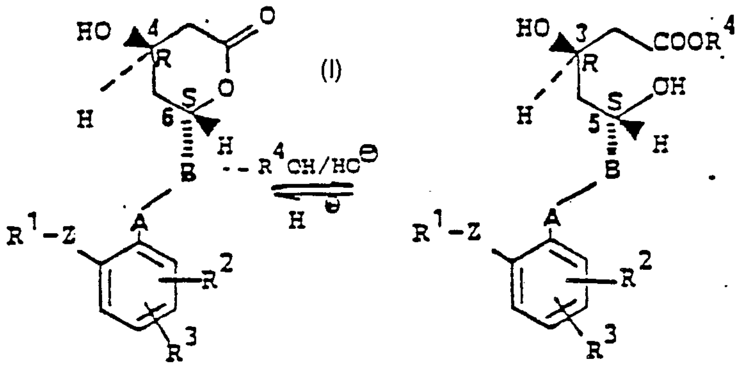


(I) (Ia)

in which

A-B denotes a radical of the formula —CH=CH— or —$CH_2$—$CH_2$—,

Z denotes a —$CH_2$— or —$CH_2$—$CH_2$— group,

$R^1$ denotes a cycloaliphatic hydrocarbon radical which has 3 to 7 carbon atoms and is optionally substituted with 1 or 2 methyl groups,

$R^2$ and $R^3$ denote hydrogen, halogen, trifluoromethyl, alkyl or alkoxy, each having 1-6 carbon atoms,

$R^4$ denotes hydrogen or a straight-chain or branched $C_1$-$C_5$-alkyl radical, benzyl, benzyl which is substituted 1-2 times by $C_1$-$C_4$-alkyl or halogen, alkali metal or ammonium ($NH_4^+$) or ammonium ion which is substituted with $C_1$-$C_4$-alkyl or hydroxy-$C_1$-$C_4$-alkyl.

2. A compound as claimed in claim 1, wherein, in formula I and Ia,

$R^1$ is cyclopentyl or cyclohexyl

$R^2$ and $R^3$ denote hydrogen, halogen, trifluoromethyl, alkyl or alkoxy, each having 1 to 4 carbon atoms and

$R^4$ represents hydrogen, methyl, ethyl, isopropyl, isobutyl, benzyl, sodium, potassium, ammonium or trishydroxymethylmethylamine.

3. A compound as claimed in claim 1, wherein, in formula I and formula Ia

$R^1$ denotes cyclopentyl or cyclohexyl

$R^2$ and $R^3$ denote hydrogen, 2-methyl, 2-trifluoromethyl, 2,4-dimethyl, 2-methyl-4-chloro, 2-chloro-4-methyl, 2,4-bistrifluoromethyl, 2-ethyl, 2-isopropyl, 2-isobutyl, 2-chloro, 2-fluoro, 2-bromo, 2,4-dichloro, 2,4-difluoro, 2-methoxy, 4-methoxy, and 2,4-dimethoxy, and

$R^4$ represents hydrogen, methyl, ethyl, benzyl, sodium, potassium, ammonium or trishydroxymethylmethylamine.

4. A process for the preparation of compounds of the formulae I and Ia, which comprises

a) reaction of the phosphonium salts of the formula II

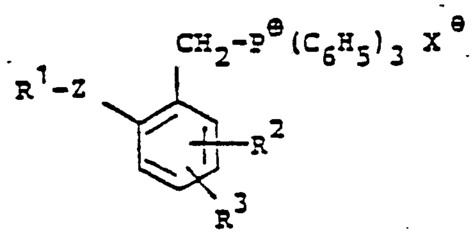

(II)

in which $R^1$, $R^2$, $R^3$ and Z have the meaning indicated for formula I, and X is Cl, Br or I, with the chiral aldehyde of the formula III

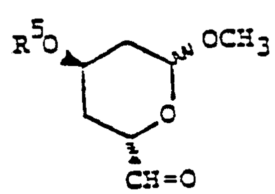

(III)

in which $R^5$ denotes a protective group which is stable to bases and weak acids, to give a compound of the formula IV

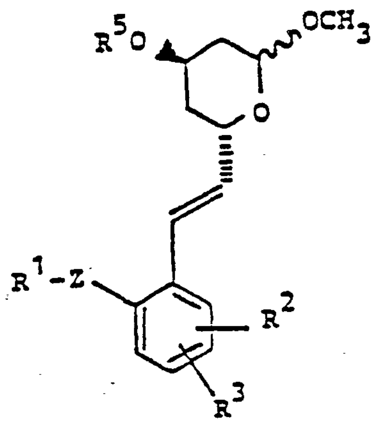

(IV)

in which $R^1$, $R^2$, $R^3$ and Z have the meaning given for formula I, $R^5$ has the meaning given for formula III (and A-B represents the (—CH=CH—) group),

b) acid hydrolysis of the methyl acetal group in a compound of the general formula IV to give a lactol of the formula V

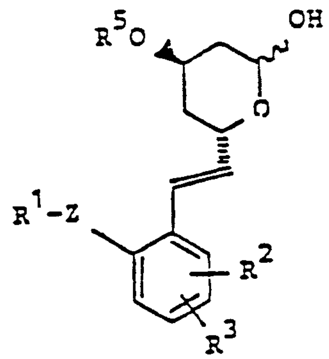

(V)

in which $R^1$, $R^2$, $R^3$ and Z have the meaning given for formula I, $R^5$ has the meaning given for formula III (and A-B represents the (—CH=CH—) group),

c) oxidation of the compound of the general formula V to give a lactone of the formula VI

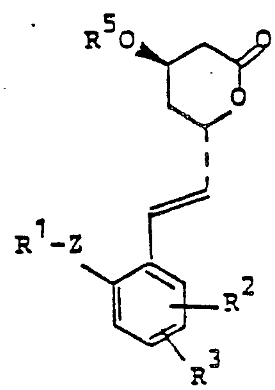

(VI)

in which $R^1$, $R^2$ and $R^3$ and Z have the meaning given for formula I, $R^5$ has the meaning given for formula III (and A-B represents the (—CH=CH—) group),

d) elimination of the protective group $R^5$ in a compound of the general formula VI to give a compound of the formula I in which $R^1$, $R^2$, $R^3$ and Z have the meanings indicated for formula I, and A-B represents the (—CH=CH—) group, where appropriate hydrogenation of a compound of the general formula I in which A-B represents a (—CH=CH—) group to give a compound of the general formula I in which A-B represents a ($—CH_2—CH_2—$) group, it also being possible to carry out the hydrogenation on the compounds of the formulae IV, V or VI to give corresponding compounds in which A-B represents the ($—CH_2—CH_2—$) group, where appropriate conversion of a hydroxy lactone I into the corresponding free hydroxy acids Ia or their salts, or, where appropriate, preparation of the corresponding ester from the free hydroxy acids Ia or from the hydroxy lactone I.

5. A pharmaceutical product which contains a compound as claimed in claim 1.

6. The use of compounds as claimed in claim 1 for the prophylaxis and therapy of arteriosclerosis and hypercholesterolemia.

7. A compound of the formula IV

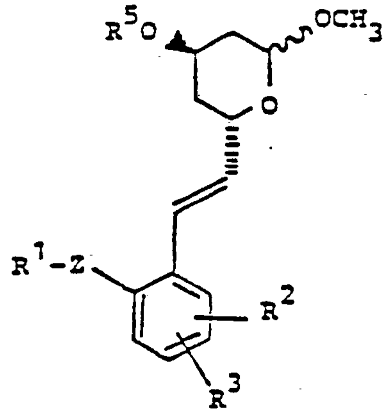

(IV)

in which $R^1$, $R^2$, $R^3$ and Z have the meanings indicated for formula I and $R^5$ has the meanings indicated for formula III.

8. A compound of the formula V

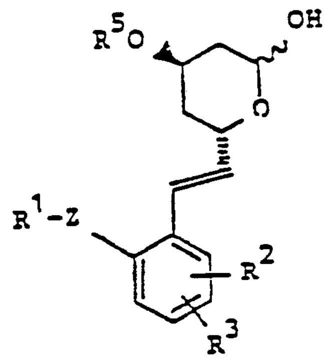

(V)

in which $R^1$, $R^2$, $R^3$ and Z have the meanings indicated for formula I and $R^5$ has the meanings indicated for formula III.

9. A compound of the formula VI

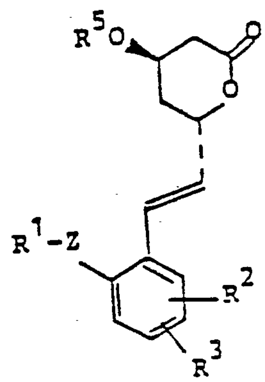

(VI)

in which $R^1$, $R^2$, $R^3$ and Z have the meanings indicated for formula I and $R^5$ has the meanings indicated for formula III.

**Claims** (for the Contracting State AT)

1. A process for the preparation of 3-demethylmevalonic acid derivatives of the formula I (δ-lactone) and Ia (corresponding dihydroxy carboxylic acid derivative)

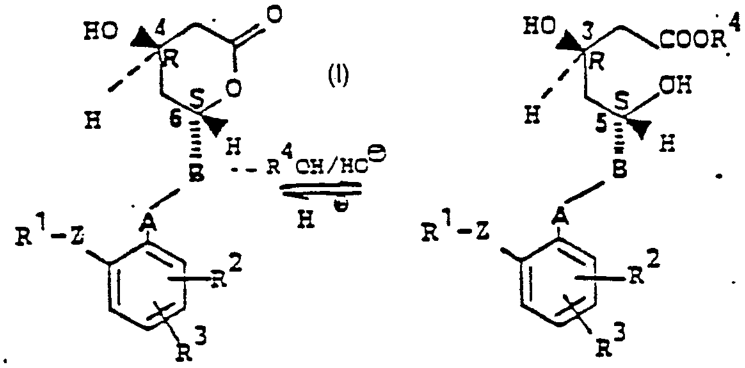


(I) (Ia)

in which

A-B denotes a radical of the formula $—CH=CH—$ or $—CH_2—CH_2$

Z denotes a $—CH_2—$ or $—CH_2—CH_2—$ group,

$R^1$ denotes a cycloaliphatic hydrocarbon radical which has 3 to 7 carbon atoms and is optionally substituted with 1 or 2 methyl groups,

$R^2$ and $R^3$ denote hydrogen, halogen, trifluoromethyl, alkyl or alkoxy, each having 1-6 carbon atoms,

$R^4$ denotes hydrogen or a straight-chain or branched $C_1$-$C_5$-alkyl radical, benzyl, benzyl which is substituted 1-2 times by $C_1$-$C_4$-alkyl or halogen, alkali metal or ammonium ($NH_4^+$) or ammonium ion which is substituted with $C_1$-$C_4$-alkyl or hydroxy-$C_1$-$C_4$-alkyl, which comprises

a) reaction of the phosphonium salts of the formula II

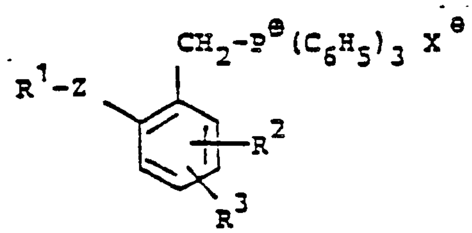

(II)

in which $R^1$, $R^2$, $R^3$ and Z have the meaning indicated for formula I, and X is Cl, Br or I, with the chiral aldehyde of the formula III

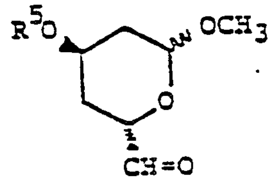

(III)

in which $R^5$ denotes a protective group which is stable to bases and weak acids, to give a compound of the formula

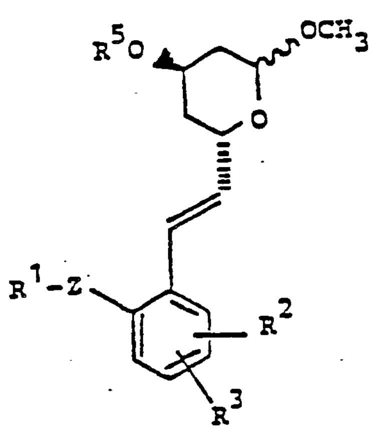

(IV)

in which $R^1$, $R^2$, $R^3$ and Z have the meaning given for formula I, $R^5$ has the meaning given for formula III (and A-B represents the ($—CH=CH—$) group),

b) acid hydrolysis of the methyl acetal group in a compound of the general formula IV to give a lactol of the formula V

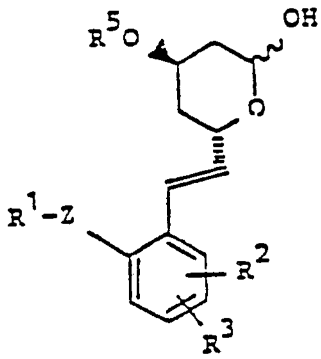

(V)

in which $R^1$, $R^2$, $R^3$ and Z have the meaning given for formula I, $R^5$ has the meaning given for formula III (and A-B represents the (—CH=CH—) group),

c) oxidation of the compound of the general formula V to give a lactone of the formula VI

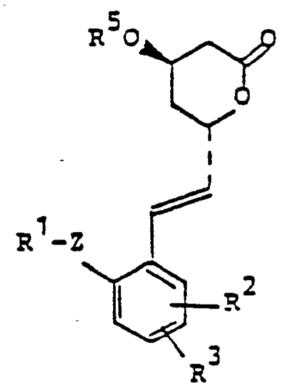

(VI)

in which $R^1$, $R^2$ and $R^3$ and Z have the meaning given for formula I, $R^5$ has the meaning given for formula III (and A-B represents the (—CH=CH—) group),

d) elimination of the protective group $R^5$ in a compound of the general formula VI to give a compound of the formula I in which $R^1$, $R^2$, $R^3$ and Z have the meanings indicated for formula I, and A-B represents the (—CH=CH—) group, where appropriate hydrogenation of a compound of the general formula I in which A-B represents a (—CH=CH—) group to give a compound of the general formula I in which A-B represents a ($—CH_2—CH_2—$) group, it also being possible to carry out the hydrogenation on the compounds of the formulae IV, V or VI to give corresponding compounds in which A-B represents the ($—CH_2—CH_2—$) group, where appropriate conversion of a hydroxy lactone I into the corresponding free hydroxy acids Ia or their salts, or, where appropriate, preparation of the corresponding ester from the free hydroxy acids Ia or from the hydroxy lactone I.

2. The process as claimed in claim 1, in which is prepared a compound of the formula I or Ia in which

$R^1$ is cyclopentyl or cyclohexyl,

$R^2$ and $R^3$ denote hydrogen, halogen, trifluoromethyl, alkyl or alkoxy, each having 1 to 4 carbon atoms and

$R^4$ represents hydrogen, methyl, ethyl, isopropyl, isobutyl, benzyl, sodium, potassium, ammonium or trishydroxymethylmethylamine.

3. The process as claimed in claim 1, in which is prepared a compound of the formula I or formula Ia in which

$R^1$ denotes cyclopentyl or cyclohexyl,

$R^2$ and $R^3$ denote hydrogen, 2-methyl, 2-trifluoromethyl, 2,4-dimethyl, 2-methyl-4-chloro, 2-chloro-4-methyl, 2,4-bistrifluoromethyl, 2-ethyl, 2-isopropyl, 2-isobutyl, 2-chloro, 2-fluoro, 2-bromo, 2,4-dichloro, 2,4-difluoro, 2-methoxy, 4-methoxy and 2,4-dimethoxy and

$R^4$ represents hydrogen, methyl, ethyl, benzyl, sodium, potassium, ammonium or trishydroxymethylmethylamine.

4. A 3-demethylmevalonic acid derivative of the formula I or Ia as claimed in claim 1, obtainable by the process as claimed in claim 1.

5. A process for the preparation of a pharmaceutical product, which comprises a compound obtainable as claimed in claim 1 being converted into a suitable form for administration.

6. The process as claimed in claim 1, wherein the intermediate of the formula IV

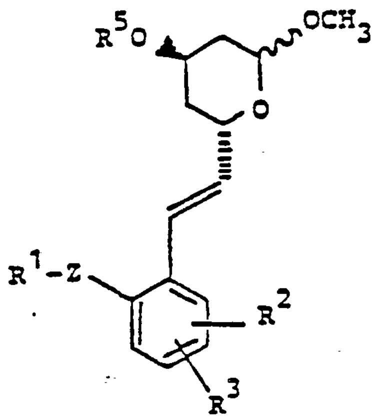

(IV)

in which $R^1$, $R^2$, $R^3$ and Z have the meanings indicated for formula I and $R^5$ has the meanings indicated for formula III, is isolated.

7. The process as claimed in claim 1, wherein the intermediate of the formula V

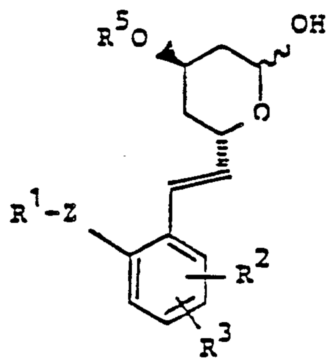

(V)

in which $R^1$, $R^2$, $R^3$ and Z have the meanings indicated for formula I and $R^5$ has the meanings indicated for formula III, is isolated.

8. The process as claimed in claim 1, wherein the intermediate of the formula VI

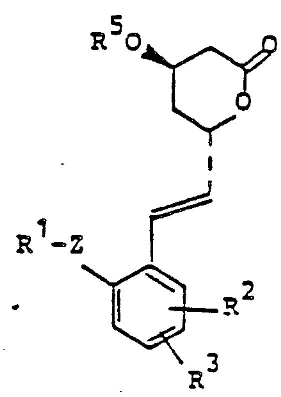

(VI)

in which $R^1$, $R^2$, $R^3$ and Z have the meanings indicated for formula I and $R^5$ has the meanings indicated for formula III, is isolated.

**Revendications** (pour les Etats contractants : BE, CH, DE, FR, GB, IT, LI, LU, NL, SE)

1. Dérivés de l'acide 3-desméthyl-mévalonique de formule I (δ-lactone) ou Ia (dérivé correspondant de l'acide dihydroxycarboxylique)

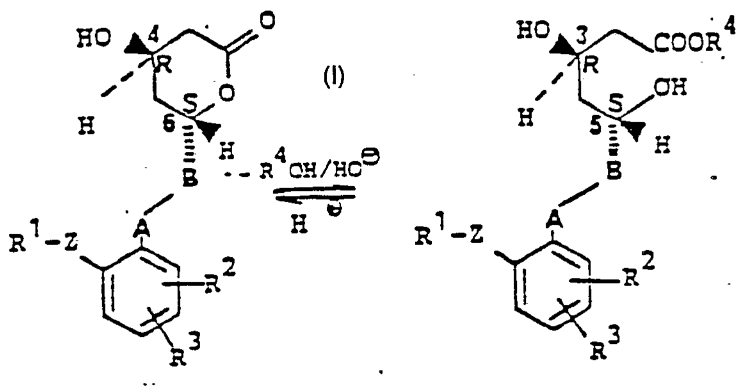


(I) (Ia)

dans lesquelles formules

A-B représente un radical de formule —CH=CH— ou $—CH_2—CH_2—$,

Z représente un groupe $—CH_2—$ ou $—CH_2—CH_2—$,

$R^1$ représente un radical hydrocarboné cycloaliphatique ayant de 3 à 7 atomes de carbone, qui est éventuellement substitué par un ou deux groupes méthyle,

$R^2$, $R^3$ représentent un atome d'hydrogène ou d'halogène, le groupe trifluorométhyle ou un groupe alkyle ou alcoxy ayant chacun de 1 à 6 atomes de carbone,

$R^4$ représente un atome d'hydrogène ou un radical alkyle en $C_1$-$C_5$ à chaîne droite ou ramifiée, benzyle, benzyle une ou deux fois substitué par un atome d'halogène ou par un groupe alkyle en $C_1$-$C_4$, un atome de métal alcalin ou l'ion ammonium ($NH_4^+$) ou un ion ammonium substitué par un groupe alkyle en $C_1$-$C_4$ ou hydroxyalkyle en $C_1$-$C_4$.

2. Composés selon la revendication 1, caractérisés en ce que, dans la formule I ou Ia,

$R^1$ est le radical cyclopentyle ou cyclohexyle,

$R^2$, $R^3$ représentent un atome d'hydrogène ou d'halogène, le groupe trifluorométhyle ou un groupe alkyle ou alcoxy ayant chacun de 1 à 4 atomes de carbone, et

$R^4$ représente un atome d'hydrogène ou de sodium ou de potassium, le groupe méthyle, éthyle, isopropyle, isobutyle, benzyle, l'ion ammonium ou le groupe tris-hydroxyméthylméthylamino.

3. Composés selon la revendication 1, caractérisés en ce que, dans la formule I ou Ia,

$R^1$ représente le radical cyclopentyle ou cyclohexyle,

$R^2$, $R^3$ représentent un atome d'hydrogène, le groupe 2-méthyle, 2-trifluorométhyle, 2,4-diméthyle, 2-méthyl-4-chloro, 2-chloro-4-méthyle, 2,4-bis-trifluorométhyle, 2-éthyle, 2-isopropyle, 2-isobutyle, 2-chloro, 2-fluoro, 2-bromo, 2,4-dichloro, 2,4-difluoro, 2-méthoxy, 4-méthoxy, 2,4-diméthoxy.

$R^4$ représente un atome d'hydrogène, de sodium ou de potassium ou le groupe méthyle, éthyle, benzyle, l'ion ammonium ou le groupe tris-hydroxyméthyl-méthylamino.

4. Procédé pour la préparation de composés de formules I et Ia, caractérisé en ce que

a) on fait réagir les sels de phosphonium de formule II

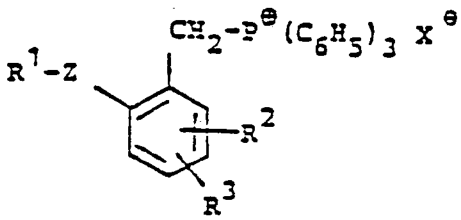

(II)

dans laquelle $R^1$, $R^2$, $R^3$ et Z ont les significations données à propos de la formule I, et X est Cl, Br, I, avec l'aldéhyde chiral de formule III

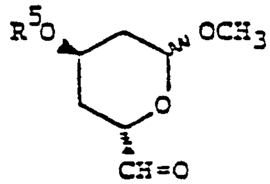

(III)

dans laquelle $R^5$ représente un groupe protecteur stable vis-à-vis des bases et des acides faibles, pour aboutir à un composé de formule

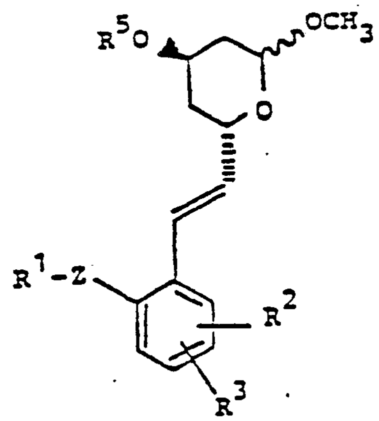

(IV)

dans laquelle $R^1$, $R^2$, $R^3$ et Z ont les significations données à propos de la formule I, $R^5$ a la signification donnée à propos de la formule III [et AB représente le groupe (—CH=CH—)],

b) on soumet à une hydrolyse acide la fonction méthylacétal dans un composé de formule générale IV, pour aboutir à un lactol de formule V

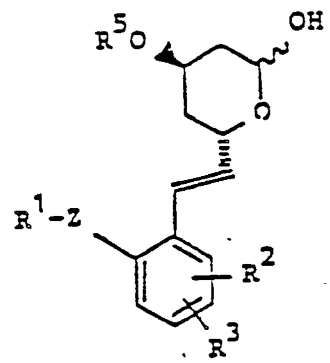

(V)

dans laquelle $R^1$, $R^2$, $R^3$ et Z ont les significations données à propos de la formule I, $R^5$ a la signification donnée à propos de la formule III [et AB représente le groupe (—CH=CH—)],

c) on oxyde le composé de formule générale V pour aboutir à une lactone de formule VI

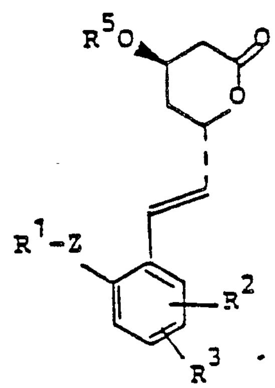

(VI)

dans laquelle $R^1$, $R^2$, $R^3$ et Z ont les significations données à propos de la formule I, $R^5$ a la signification donnée à propos de la formule III [et AB représente le groupe (—CH=CH—)],

d) on élimine le groupe protecteur $R^5$ dans un composé de formule générale VI, pour aboutir à un composé de formule I dans lequel $R^1$, $R^2$, $R^3$ et Z ont les significations données à propos de la formule I et A-B représente le groupe (—CH=CH—), éventuellement on soumet à une hydrogénation un composé de formule générale I dans lequel A-B représente le groupe (—CH=CH—), pour aboutir à un composé de formule générale I dnas lequel A-B représente le groupe (—CH=$CH_2$—), l'hydrogénation pouvant également être effectuée sur les composés de formules IV, V ou VI, pour donner les composés correspondants dans lesquels A-B représente le groupe (—$CH_2$=$CH_2$—), éventuellement on convertit une hydroxylactone I en les acides hydroxycarboxyliques la libres correspondants ou en leurs sels, ou

éventuellement on prépare les esters correspondants à partir des acides hydroxycarboxyliques Ia libres ou à partir de l'hydroxylactone I.

5. Composition pharmaceutique, caractérisée en ce qu'elle contient un composé selon la revendication 1.

6. Utilisation de composés selon la revendication 1, pour la prophylaxie et le traitement de l'artériosclérose et de l'hypercholestérolémie.

7. Composés de formule IV

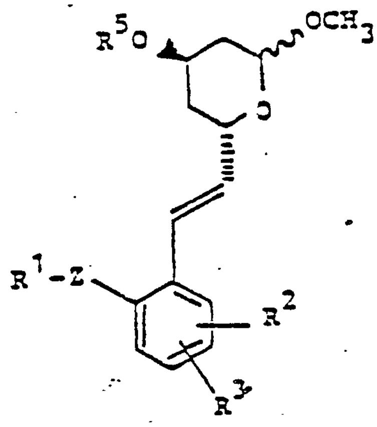

(IV)

dans laquelle $R^1$, $R^2$, $R^3$ et Z ont les significations données à propos de la formule I, et $R^5$ a la signification donnée à propos de la formule III.

8. Composés de formule V

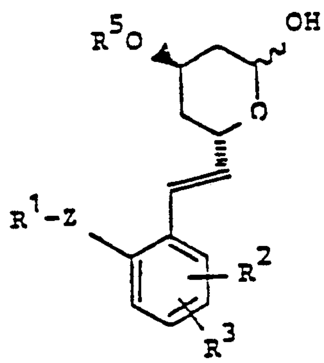

(V)

dans laquelle $R^1$, $R^2$, $R^3$ et Z ont les significations données à propos de la formule I, et $R^5$ a la signification donnée à propos de la formule III.

9. Composés de formule VI

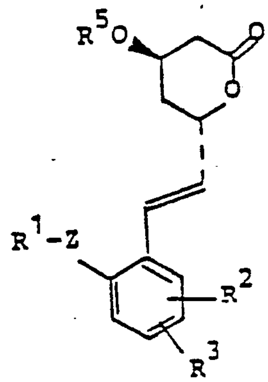

(VI)

dans laquelle $R^1$, $R^2$, $R^3$ et Z ont les significations données à propos de la formule I, et $R^5$ a la signification donnée à propos de la formule III.

**Revendications** (pour l'Etat contractant AT)

1. Procédé pour la préparation de dérivés de l'acide 3-desméthyl-mévalonique de formule I (δ-lactone) ou la (dérivé correspondant de l'acide dihydroxycarboxylique)

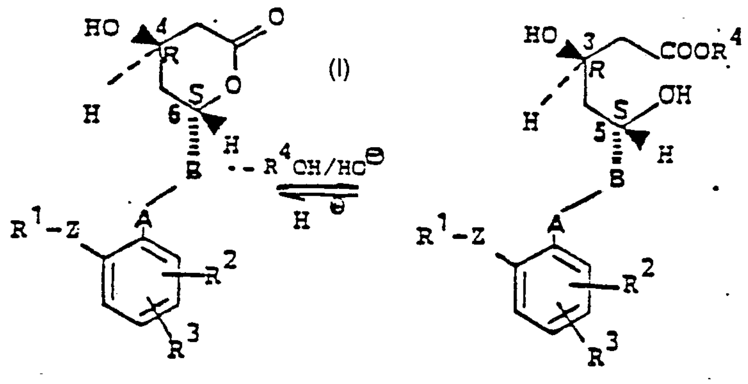

(I)

(Ia)

dans lesquelles formules

A-B représente un radical de formule —CH=CH— ou —$CH_2$—$CH_2$—,

Z représente un groupe —$CH_2$— ou —$CH_2$—$CH_2$—,

$R^1$ représente un radical hydrocarboné cycloaliphatique ayant de 3 à 7 atomes de carbone, qui est éventuellement substitué par un ou deux groupes méthyle,

$R^2$, $R^3$ représentent un atome d'hydrogène ou d'halogène, le groupe trifluorométhyle ou un groupe alkyle ou alcoxy ayant chacun de 1 à 6 atomes de carbone,

$R^4$ représente un atome d'hydrogène ou un radical alkyle en $C_1$-$C_5$ à chaîne droite ou ramifiée, benzyle, benzyle une ou deux fois substitué par un atome d'halogène ou par un groupe alkyle en $C_1$-$C_4$, un atome de métal alcalin ou l'ion ammonium ($NH_4^+$) ou un ion ammonium substitué par un groupe alkyle en $C_1$-$C_4$ ou hydroxyalkyle en $C_1$-$C_4$, caractérisé en ce que

a) on fait réagir les sels de phosphonium de formule II

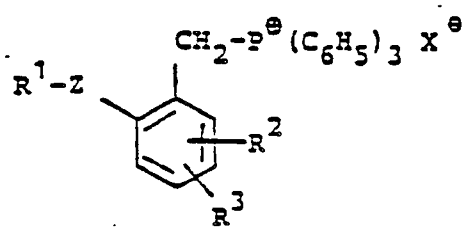

(II)

dans laquelle $R^1$, $R^2$, $R^3$ et Z ont les significations données à propos de la formule I, et X est Cl, Br, I, avec l'aldéhyde chiral de formule III

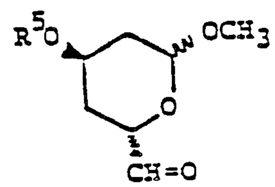

(III)

dans laquelle $R^5$ représente un groupe protecteur stable vis-à-vis des bases et des acides faibles, pour aboutir à un composé de formule

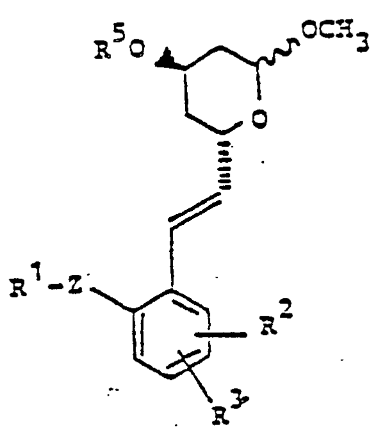

(IV)

dans laquelle $R^1$, $R^2$, $R^3$ et Z ont les significations données à propos de la formule I, $R^5$ a la signification donnée à propos de la formule III [et AB représente le groupe (—CH=CH—)],

b) on soumet à une hydrolyse acide la fonction méthylacétal dans un composé de formule générale IV, pour aboutir à un lactol de formule V

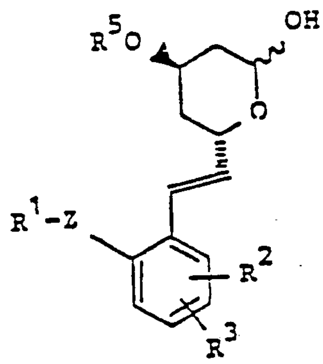

(V)

dans laquelle $R^1$, $R^2$, $R^3$ et Z ont les significations données à propos de la formule I, $R^5$ a la signification donnée à propos de la formule III [et AB représente le groupe (—CH=CH—)],

c) on oxyde le composé de formule générale V, pour aboutir à une lactone de formule VI

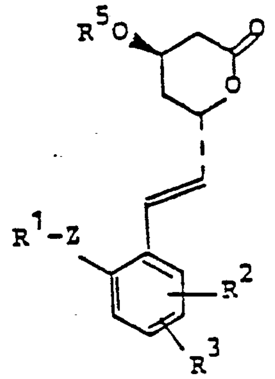

(VI)

dans laquelle $R^1$, $R^2$, $R^3$ et Z ont les significations données à propos de la formule I, $R^5$ a la signification donnée à propos de la formule III [et AB représente le groupe (—CH=CH—)],

d) on élimine le groupe protecteur $R^5$ dans un composé de formule générale VI, pour aboutir à un composé de formule I dans lequel $R^1$, $R^2$, $R^3$ et Z ont les significations données à propos de la formule I et A-B représente le groupe (—CH=CH—), éventuellement on soumet à une hydrogénation un composé de formule générale I dans lequel A-B représente le groupe (—CH=CH—), pour aboutir à un composé de formule générale I dans lequel A-B représente le groupe ($—CH_2—CH_2—$), l'hydrogénation pouvant également être effectuée sur les composés de formules IV, V ou VI, pour donner les composés correspondants dans lesquels A-B représente le groupe ($—CH_2—CH_2—$), éventuellement on convertit une hydroxylactone I en les acides hydroxycarboxyliques Ia libres correspondants ou en leurs sels, ou éventuellement on prépare les esters correspondants à partir des acides hydroxycarboxyliques Ia libres ou à partir de l'hydroxylactone I.

2. Procédé selon la revendication 1, caractérisé en ce que l'on prépare un composé de formule I ou Ia dans lequel

$R^1$ est le radical cyclopentyle ou cyclohexyle,

$R^2$, $R^3$ représentent un atome d'hydrogène ou d'halogène, le groupe trifluorométhyle ou un groupe alkyle ou alcoxy ayant chacun de 1 à 4 atomes de carbone, et

$R^4$ représente un atome d'hydrogène ou de sodium ou de potassium, le groupe méthyle, éthyle, isopropyle, isobutyle, benzyle, l'ion ammonium ou le groupe tris-hydroxyméthylméthylamino.

3. Procédé selon la revendication 1, caractérisé en ce que l'on prépare un composé de formule I ou Ia dans lequel

$R^1$ représente le radical cyclopentyle ou cyclohexyle,

$R^2$, $R^3$ représentent un atome d'hydrogène, le groupe 2-méthyle, 2-trifluorométhyle, 2,4-diméthyle, 2-méthyl-4-chloro, 2-chloro-4-méthyle, 2,4-bis-trifluorométhyle, 2-éthyle, 2-isopropyle, 2-isobutyle, 2-chloro, 2-fluoro, 2-bromo, 2,4-dichloro, 2,4-difluoro, 2-méthoxy, 4-méthoxy, 2,4-diméthoxy.

$R^4$ représente un atome d'hydrogène, de sodium ou de potassium ou le groupe méthyle, éthyle, benzyle, l'ion ammonium ou le groupe tris-hydroxyméthyl-méthylamino.

4. Dérivés de l'acide 3-desméthyl-mévalonique de formule I ou la selon la revendication 1, pouvant être obtenus par le procédé selon la revendication 1.

5. Procédé pour la fabrication d'une composition pharmaceutique, caractérisé en ce que l'on met sous une forme pharmaceutique appropriée un composé pouvant être obtenu selon la revendication 1.

6. Procédé selon la revendication 1, caractérisé en ce que l'on isole le produit intermédiaire de formule IV

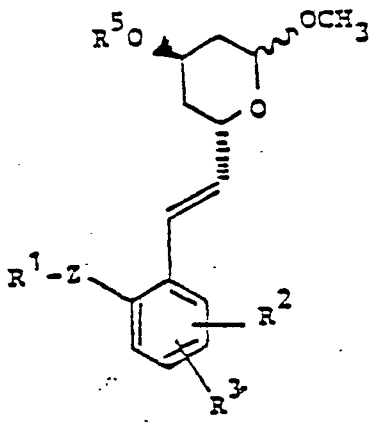

(IV)

dans laquelle $R^1$, $R^2$, $R^3$ et Z ont les significations données à propos de la formule I, et $R^5$ a la signification donnée à propos de la formule III.

7. Procédé selon la revendication 1, caractérisé en ce que l'on isole le produit intermédiaire de formule V

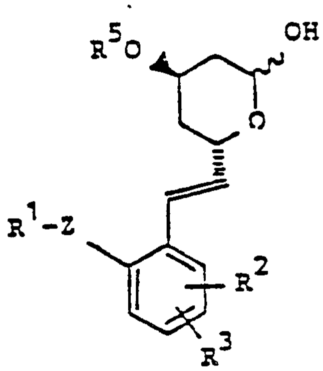

(V)

dans laquelle $R^1$, $R^2$, $R^3$ et Z ont les significations données à propos de la formule I, et $R^5$ a la signification donnée à propos de la formule III.

8. Procédé selon la revendication 1, caractérisé en ce que l'on isole le produit intermédiaire de formule VI

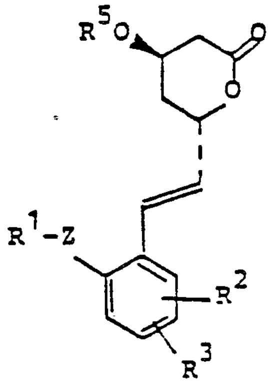

(VI)

dans laquelle $R^1$, $R^2$, $R^3$ et Z ont les significations données à propos de la formule I, et $R^5$ a la signification donnée à propos de la formule III.